# EUROPEAN PATENT SPECIFICATION

(11) **EP 1 773 779 B1**
(45) Date of publication and mention of the grant of the patent: **04.12.2013**
(21) Application number: 05764252.2
(22) Date of filing: 15.07.2005
(51) Int. Cl.: C07D 223/04, A61P 29/00, A61K 31/495, A61K 31/465, A61K 31/4439, A61K 31/439, A61K 31/221, A61K 31/551, C07D 295/037, C07D 243/08

(54) **NICOTINIC RECEPTOR AGONISTS FOR THE TREATMENT OF INFLAMMATORY DISEASES**
NICOTINREZEPTORAGONISTEN ZUR BEHANDLUNG ENTZÜNDLICHER KRANKHEITEN
AGONISTES DU RECEPTEUR DE NICOTINE POUR LE TRAITEMENT DE MALADIES INFLAMMATOIRES

(30) Priority: 15.07.2004 US 890987
(43) Date of publication of application: 18.04.2007
(73) Proprietor: Université Laval, Laval, QC G1K 7P4 (CA)
(72) Inventor: CORMIER, Yvon, Neuville, Québec G0A 2R0 (CA); ISRAEL-ASSAYAG, Evelyne, Sainte-Foy, Québec G1X 3J8 (CA); BLANCHET, Marie-Renée, St-Pierre, Ile d'Orléans, Québec G0A 4E0 (CA); GAUDREAULT, René C., St-Nicolas, Québec G7A 2N3 (CA); LABRIE, Philippe, Quebec, Québec G1M 4C6 (CA)
(74) Representative: Fiorucci, Hélène
(86) International application number: PCT/CA2005/001120
(87) International publication number: WO 2006/005195

(56) References cited:
- EP-A1- 1 462 091
- CA-A1- 2 382 567
- CA-A1- 2 441 096
- US-A- 3 402 039
- ROMANELLI, M. ET AL.: "Structure-affinity relationships of a unique nicotinic ligand: N1-dimethyl-N4-phenylpiperazinium iodide (DMPP)." J. MED. CHEM., vol. 44, 2001, pages 3946-3955, XP002541221
- GREEN, A. L. ET AL.: "Nicotinic stimulant action of some tolyl and xylyl analogues of 1,1-dimethyl-4-phenylpiperazinium (DMPP)." BRITISH J. PHARMAC., vol. 43, 1971, pages 370-378, XP002541222
- DATABASE REGISTRY [Online] XP002997187 Database accession no. 54-77-3

## Description

### CROSS-REFERENCE TO RELATED APPLICATIONS

Continuation-in-part of Ser. No.10/890,987 filed July 15,2004 that is a continuation-in-part of Ser. No. 10/469,999, filed February 24, 2004 that is a National Stage application of PCT/CA02/00412 filed March 25, 2002.

### BACKGROUND OF THE INVENTION

### a) Field of the Invention

The present invention relates to the treatment of inflammatory diseases, including a variety of pulmonary diseases, through the use or administration of nicotinic receptor agonists or analogs and derivatives thereof.

### b) Description of Prior Art

Although a normal man or woman breathes more than one cubic meter of air every hour, our lung defense mechanisms usually deal with the large quantities of particles, antigens, infectious agents and toxic gases and fumes that are present in inhaled air. The interaction of these particles with the immune system and other lung defense mechanisms results in the generation of a controlled inflammatory response which is usually protective and beneficial. In general, this process regulates itself in order to preserve the integrity of the airway and alveolar epithelial surfaces where gas exchange occurs. In some cases, however, the inflammatory response cannot be regulated and the potential for tissue injury is increased. Depending on the type of environmental exposure, genetic predisposition, and a variety of ill-defined factors, abnormally large numbers of inflammatory cells can be recruited at different sites of the respiratory system, resulting in illness or disease.

The inflammatory response to inhaled or intrinsic stimuli is characterized by a non-specific increase in the vascular permeability, the release of inflammatory and chemotactic mediators including histamine, eicosanoids, prostaglandins, cytokines and chemokines. These mediators modulate the expression and engagement of leukocyte-endothelium cell adhesion molecules allowing the recruitment of inflammatory cells present in blood.

A more specific inflammatory reaction involves the recognition and the mounting of an exacerbated, specific immune response to inhaled antigens. This reaction is involved in the development of asthma, hypersensitivity pneumonitis (HP) and possibly sarcoidosis. Dysregulation in the repair mechanisms following lung injury may contribute to fibrosis and loss of function in asthma, pulmonary fibrosis, chronic obstructive pulmonary disease (COPD), and chronic HP.

It was previously reported that the incidence of HP is much lower among current smokers than in non-smokers (1-4). Sarcoidosis is also less frequent in smokers than in non smokers (5, 6). The mechanisms underlying the beneficial effects of cigarette smoking on the development of HP and other inflammatory diseases are still unknown but may be linked to the immunomodulatory effect of nicotine. There are clinical observations of asthma *de novo* or exacerbation after smoking cessation. Proof of this is difficult to obtain and any protective effects of nicotine in the prevention or treatment of asthma are likely overwhelmed by the negative effects of tobacco smoke with its thousands of constituents.

The protective effect of smoking has also been reported in other diseases, the most studied being ulcerative colitis, an inflammatory intestinal disease (7, 8). Nicotine has been successfully used in the treatment of this disease (9, 10). Other studies have looked at the possible therapeutic value of nicotine in the treatment of Alzheimer's disease and Parkinson's disease. (11, 12).

Nicotinic receptors are pentamers made up of five polypeptide subunits which act as ligand-gated ions channels. When the ligand binds to the receptor, a conformational change in the polypeptide occurs, opening a central channel that allows sodium ion to move from the extracellular fluid into the cytoplasm. Four types of subunits have been identified: α, β, y and δ. The receptor can consist of any combination of these four types of subunits (13). Recent work has shown that alveolar macrophages (AM) can express the α-7 subunit (14), while bronchial epithelial cells express the α-3, α-5 and α-7 subunits (15), and lymphocytes the α-2, α-5, α-7, β-2 and β-4 subunits (14). Fibroblasts (16) and airway smooth muscles cells (17) also express these receptors. Therefore, resident pulmonary cells (AM, dendritic cells, epithelial cells, fibroblasts, etc.) and those recruited in inflammatory diseases (lymphocytes, polymorphonuclear cells) express nicotinic receptors.

Nicotinic receptor activation in lymphocytes affects the intracellular signalization, leading to incomplete activation of the cell. In fact, nicotine treatment upregulates protein kinase activity, which in turn upregulates phospholipase A2 (PLA2) activity. PLA2 is responsible for cleaving phosphoinositol-2-phosphate (PIP2) into inositol-3-phosphate (IP3) and diacylglycerol (DAG) (18, 19). The continuous presence of IP3 in the cell would appear to result in the desensitization of calcium stores, leading to their depletion (19). This observation could explain the fact that nicotine-treated lymphocytes do not release enough calcium into the cytoplasm to activate transcription factors such as NFk-B (20).

Nicotine, the major pharmacological component of cigarette smoke, is one of the best known nicotinic receptor agonists (21). This natural substance has well defined anti-inflammatory and immunosuppressive properties (22), and may have anti-fibrotic properties (23). Exposure of animals to smoke from cigarettes with high levels of nicotine is more immunosuppressive than that from low-nicotine cigarettes (24). Moreover, treatment of rats with nicotine inhibits the specific antibody response to antigens and induces T cell anergy (25). Although they are increased in number, AM from smokers show a decreased ability to secrete inflammatory cytokines in response to endotoxins ((20, 25, 26)) and nicotine seems to be the responsible component of this inhibition (26). One study also showed that peripheral blood lymphocytes from smokers express higher levels of FAS ligand (FASL) and that nicotine increases FASL expression on lymphocytes from non-smokers, indicating that nicotine may affect cell apoptosis (27). Nicotine was also shown to have an inhibitory effect on the proliferation and extracellular matrix production of human gingival fibroblasts *in vitro* (23). Of interest, nicotine treatment seems to up-regulate the expression of nicotinic receptors (28). Nicotine itself is a safe substance that does not seem to have any long term side effects (48-49). Smoke-related diseases of the lungs, heart and arteries are not caused by nicotine but by the thousands of other chemicals present in the inhaled smoke. The main problem is that nicotine crosses the blood-brain barrier, inducing addiction. The harmful effects of cigarette smoking are obvious. Although nicotine is not responsible for the toxic effects of cigarette smoking, the association remains.

Nicotinic agonists may down-regulate T cell activation, indeed, nicotine has been shown to affect T cell expression of the co-stimulatory molecules CD28 and CTLA4 (29).

The B7/CD28/CTLA4 co-stimulatory pathway plays a key regulatory role in T-cell activation and homeostasis (30, 31). Two signaling pathways are involved. A positive signal involves the engagement of B7 (CD80 /CD86) molecules with T cell CD28 receptors which results in the potentiation of T cell responses (proliferation, activation, cytokine expression, and survival) (32). A negative signal involves B7 interactions with CTLA4 on activated T cells, leading to a downmodulation of T cell responses (33, 34). The balance between CD28 and CTLA4 derived signals may alter the outcome of T-cell activation.

In HP, it was previously reported that an upregulation of B7 molecule expression on AM in patients with active HP (35) and in murine HP (36). It was also shown that a blockade of the B7-CD28 co-stimulatory pathway in mice inhibited lung inflammation (36). These results also demonstrated that the expression of B7 molecules on AM is lower in smokers than in non-smokers and that an *in vitro* influenza virus infection is able to upregulate B7 expression in normal human AM but not in AM from smokers; whether this is due to nicotine or other substances present in cigarette smoke is unknown (35). An up-regulation of the B7 molecules has also been reported in asthma (37, 38) and sarcoidosis (39).

Epibatidine is the most potent nicotinic agonist known so far (40). It has anti-inflammatory and analgesic properties. In fact, its analgesic potential is two hundred times that of morphine (40). This molecule is also known to inhibit lymphocyte proliferation *in vitro* (41). The binding of epibatidine to the receptor is non-specific (42). Unfortunately, epibatidine has major toxic side effects mostly on the cardiovascular and the central nervous systems making it inappropriate for use as an anti-inflammatory drug to treat pulmonary diseases (40).

Dimethylphenylpiperazinium (DMPP) is a synthetic nicotinic agonist that is non-specific (13). Its potency for the receptor is about the same as nicotine, depending on the kind of cells implicated in the stimulation (43). Its advantage over nicotine and other nicotinic agonists is that its chemical configuration prevents it from crossing the blood-brain barrier, thus causing no addiction or other central nervous effects (13). The anti-inflammatory properties of DMPP are not well described. However, it has been shown that a chronic *in vivo* treatment could decrease the number of white, blood cells, decrease the cytokine production by splenocytes and decrease the activity of natural killer cells (44). The effect of DMPP on airway smooth muscle cells has also been tested. DMPP has an initial short contractive effect which is followed by a relaxing effect when the cells are in contact with the agonist for a longer period of time (45). This bronchodilatory effect may not necessarily in itself make DMPP the most useful treatment of asthma, since other potent bronchodilators are currently available on the market (B2 agonists). However, the properties of this nicotinic receptor agonist are important since this drug could be safely administered to asthmatics and COPD patients for its anti-inflammatory properties. Moreover, there is no apparent evidence that DMPP has any toxic effect on major organs such as the heart, the brain, the liver or the lungs.

Corticosteroids are potent anti-inflammatory drugs. Their systemic use causes major side effects that preclude their long-term uses whenever possible. Inhaled poorly absorbed steroids are useful to treat airway inflammation. At low doses these drugs have little or no side effects. However, higher doses increase the risks for oral candidasis, vocal cords paralysis, cataracts and osteoporosis. Inhaled steroids have no effects on lung interstitium and have no anti-fibrotic properties (57)

More recent drugs, such as anti-leukotrienes, are useful in some asthmatics (58) but have no effects in COPD and other lung diseases. These drugs have anti-inflammatory properties limited to the components of inflammation caused by leukotrienes (59). The treatment of interstitial lung disease such as IPF, Sarcoidosis, HP, and BOOP basically rests on the use of systemic corticosteroids. This treatment is effective in controlling some of the inflammation but unfortunately induces serious side effects and does not reverse underlying fibrotic changes. Immunosupressive agents such as cyclophosphamide and azathioprine are sometimes tried in severe IPF but their therapeutic values are unproven and at most, very limited (60). In essence, lung fibrosis is usually progressive and untreatable, with most IPF patients dying of this condition (61).

Despite advances in the treatment of inflammatory illnesses, including pulmonary inflammatory diseases, treatment using available drugs or agents frequently results in undesirable side effects. For example, the inflammation of COPD is apparently resistant to corticosteroids, and consequently the need for the development of new anti-inflammatory drugs to treat this condition has been recognized (46).

Similarly, while corticosteroids and other immunosuppressive medications have been routinely employed to treat pulmonary fibrosis, they have demonstrated only marginal efficacy (47).

There is thus a need for new and reliable methods of treating inflammatory diseases, including pulmonary inflammatory diseases, in a manner that alleviates their symptoms without causing side effects.

### SUMMARY OF THE INVENTION

In accordance with the present invention, there is provided compounds for use in treating inflammatory diseases. Specifically, compounds are described for use in treating pulmonary inflammatory diseases through the use or administration of an agent that binds to or modulates the function nicotinic receptor, such as nicotinic receptor agonists or analogues or derivatives thereof

More precisely, the present invention concerns:
- a compound according to any of claims 1 to 6;
- a pharmaceutical composition according to any of claim 7 to 10;
- a use of a compound in the preparation of a medicament for treating or preventing pulmonary inflammatory diseases selected from the group consisting of asthma, chronic obstructive pulmonary disease (COPD), interstitial pulmonary fibrosis (IPF), sarcoidosis, hypersensitivity pneumonitis (HP), chronic HP and bronchiolitis obliterans with organizing pneumonitis (BOOP) according to any of claims 11 to 20 ;
- a use of a compound in the preparation of a medicament for inducing airway smooth muscle relaxation and bronchodilation according to claim 21 or for inducing agonistic response in a pulmonary cell nicotinic receptor according to claim 22;
- a compound for use in treating or preventing pulmonary inflammatory diseases selected from the group consisting of asthma, chronic obstructive pulmonary disease (COPD), interstitial pulmonary fibrosis (IPF), sarcoidosis, hypersensitivity pneumonitis (HP), chronic HP and bronchiolitis obliterans with organizing pneumonitis (BOOP) according to any of claims 23 to 32;
- a compound according to any one of claims 1-6, for use in inducing airway smooth muscle relaxation and bronchodilation.
- a compound according to any one of claims 1-6, for use in inducing agonistic response in a pulmonary cell nicotinic receptor.

In one aspect, there are therefore provided compounds that modulate the function of nicotinic receptors for use in treating or preventing pulmonary inflammatory diseases.

In a further aspect, there is also provided compounds of formula: 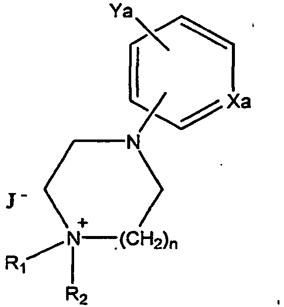 wherein R₁ and R₂ are independently lower alkyl of 1 to 10 carbon atoms,
Xa is CH or N,
Ya is one or more substituent selected from hydrogen, halogen, amidino, amido, azido, cyano, guanido, hydroxyl, nitroso, urea, sulfate, sulfite, sulfonate, sulphonamide, phosphate, phosphonate, acyl, acyloxy, alkyl of 1 to 6 carbon atoms, alkoxy of 1 to 6 carbon atoms, alkylthio of 1 to 6 carbon atoms, alkylamino of 1 to 6 carbon atoms, alkanol of 1 to 6 carbon atoms, aralkyl, aryl of 6 to 10 carbon atoms and 3 to 10 membered heterocycle
n is 2,
J is a counter ion.

In still a further aspect, there is provided a pharmaceutical composition for treating pulmonary inflammatory diseases comprising a nicotinic receptor agonist as claimed and a pharmaceutically acceptable excipient.

In a further aspect, there is provided an effective amount of a compound having the formula: 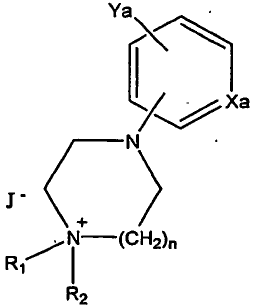 wherein R₁, R₂, Xa, Ya and J are as described herein, for use in inducing airways smooth muscle relaxation.

In another aspect, there is provided by the present invention an effective amount of a nicotinic receptor agonist as claimed for use in inducing agonistic response in a pulmonary cell nicotinic receptor.

### BRIEF DESCRIPTION OF THE DRAWINGS

The invention is illustrated but is not limited by the annexed drawings, in which:
Fig. 28 illustrates the effect of DMPP, ASM-002, ASM-003, ASM-004, and ASM-005 on TNF release
Fig. 29 illustrates the effect of DMPP, ASM-002, ASM-003, ASM-004, and ASM-005 on mouse tracheal airway smooth muscle responsiveness;
Fig. 30 illustrates the effect of ASM-002 on lung inflammation;
Fig. 31 illustrates the effects of ASM-002 on lung resistance in a mouse model of asthma;
Fig. 32 illustrates the comparative effects of ASM-002 and prednisone on lung inflammation;
Fig. 33 illustrates the effects of ASM-002 in a dog model of lung hyper-responsiveness;
Fig. 34 illustrates the muscle-relaxing properties of ASM-002 on mouse tracheas;
Fig. 35 illustrates the muscle-relaxing properties of ASM-002 on dog bronchial rings;
Fig. 36 illustrates the muscle-relaxing properties of ASM-002 on human bronchial rings;
Fig. 37 illustrates the inhibitory effects of ASM-002 on potent inflammatory mediators release by human blood cells isolated from asthmatic patients;
Fig. 38 illustrates the comparative effects of ASM-002 with DMPP and dexamethasone on TNF production by LPS-stimulated blood monocytes;
Fig. 39 illustrates the inhibition of LTC4 production by ASM-002;

### DESCRIPTION OF PREFERRED EMBODIMENTS

Other objects, advantages and features of the present invention will become more apparent upon reading the following non-restrictive description of preferred embodiments thereof, given by way of example only with reference to the accompanying drawings.

The idea of using nicotine or other nicotinic receptor agonists or analogs or derivatives thereof to treat inflammatory pulmonary disease is novel. Despite the impressive anti-inflammatory and immunosuppressive properties of nicotine and other nicotinic receptor agonists or analogs or derivatives, their usefulness in the treatment of allergic and other inflammatory lung diseases has not previously been disclosed. The drawbacks associated with cigarette are major reasons for the lack of prior interest in nicotinic agonists or analogs or derivatives thereof in the treatment of lung diseases.

The present invention thus proposes the of use nicotinic receptor agonists, such as DMPP analogs as well as derivatives thereof, to treat inflammatory lung diseases such as asthma, COPD, interstitial pulmonary fibrosis (IPF), sarcoidosis, HP, and bronchiolitis obliterans with organizing pneumonitis (BOOP). The drug could be administered orally or, depending on the specific diseases or conditions, by targeted delivery directly to the lung by aerosolisation with different and preferred vehicles in order to minimize systemic effects.

The anti-inflammatory, immunosuppressive and/or bronchodilating properties, as well as minimal side effects of nicotinic receptor agonists and analogs and derivatives thereof, make these drugs ideally suited for medical use in the treatment of a large variety of lung diseases that are characterized by bronchial or interstitial inflammation. These diseases include diseases such as asthma, COPD, IPF, sarcoidosis, HP and BOOP.

In accordance with one embodiment, the invention provides an effective amount of a compound that modulates the function of nicotinic receptors for use in treating or preventing pulmonary inflammatory diseases comprising administering.

In one embodiment, the compound for use in the method of the invention is a nicotinic receptor agonist as claimed.

In another embodiment, the compounds for use in the method of the invention are:
i) a compound having the formula: 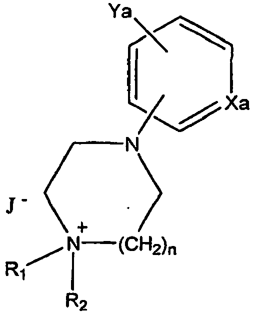 wherein R₁ and R₂ are independently lower alkyl of 1 to 10 carbon atoms,
   Xa is CH or N,
   Ya is one or more substituent selected from hydrogen, halogen, amino, amidino, amido, azido, cyano, guanido, hydroxyl, nitro, nitroso, urea, sulfate, sulfite, sulfonate, sulphonamide, phosphate, phosphonate, acyl, acyloxy, alkyl of 1 to 6 carbon atoms, alkoxy of 1 to 6 carbon atoms, alkylthio of 1 to 6 carbon atoms, alkylamino of 1 to 6 carbon atoms, alkanol of 1 to 6 carbon atoms, aralkyl, aryl of 6 to 10 carbon atoms and 3 to 10 membered heterocycle
   n is 2,
   J is a counter ion.

In a further embodiment, the compound useful in the method of the invention has the formula: 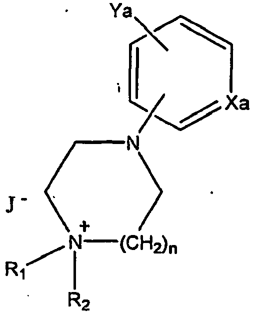 wherein R₁ and R₂ are independently alkyl of 1 to 10 carbon atoms,
Xa is CH or N,
Ya is one or more substituent selected from hydrogen, halogen, amino, amidino, amido, azido, cyano, guanido, hydroxyl, nitro, nitroso, urea, sulfate, sulfite,
sulfonate, sulphonamide, phosphate, phosphonate, acyl, acyloxy, alkyl of 1 to 10 carbon atoms, alkoxy of 1 to 10 carbon atoms, alkylthio of 1 to 10 carbon atoms, alkylamino of 1 to 10 carbon atoms, alkanol of 1 to 10 carbon atoms, aralkyl, aryl of 6 to 10 carbon atoms and 3 to 10 membered heterocycle;
n is 2,
J is a counter ion.

In still a further embodiment, R₁ and R₂ are independently optionally substituted lower alkyl of 1 to 10 carbon atoms;
Xa is CH;
Ya is one or more substituent selected from hydrogen, halogen, amino, amido, hydroxyl, alkyl of 1 to 6 carbon atoms, alkoxy of 1 to 6 carbon atoms and alkanol of 1 to 6 carbon atoms;
n is 2;
J is a halogen.

In another embodiment, the compounds for use in the method of the invention has the formula: 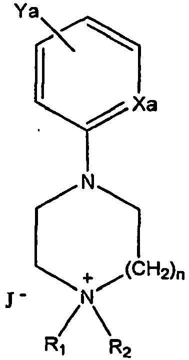 wherein R₁ and R₂ are independently optionally substituted lower alkyl of 1 to 6 carbon atoms;
Xa is CH;
Ya is one or more substituent selected from hydrogen, halogen, amino, amido, hydroxyl, alkyl of 1 to 6 carbon atoms, alkoxy of 1 to 6 carbon atoms, lower alkanol of 1 to 6 carbon atoms;
n is 2;
J is a halogen.

In an additional embodiment, R₁ and R₂ are independently selected from methyl, ethyl, n-propyl, or i-propyl;
Xa is CH;
Ya is hydrogen;
n is 2;
J is a halogen.

In an additional embodiment, the compound has the formula: 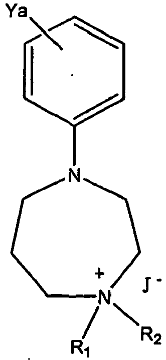 wherein R₁ and R₂ are independently selected from methyl, ethyl, n-propyl, or i-propyl;
Ya is hydrogen;
J is a halogen.

In a further embodiment, the compound for use in the method of the invention has the formula:

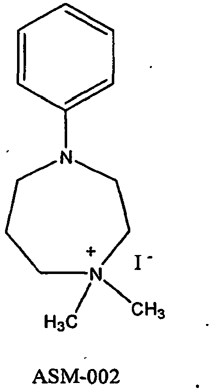

In one embodiment, the pulmonary inflammatory disease is selected from the group consisting of asthma, chronic obstructive pulmonary disease (COPD), interstitial pulmonary fibrosis (IPF), sarcoidosis, hypersensitivity pneumonitis (HP), chronic HP and bronchiolitis obliterans with organizing pneumonitis (BOOP).

In one embodiment, the pulmonary inflammatory disease is selected from the group consisting of asthma, chronic obstructive pulmonary disease (COPD), interstitial pulmonary fibrosis (IPF), sarcoidosis, hypersensitivity pneumonitis (HP) and chronic HP.

In further embodiments, the pulmonary inflammatory disease is: chronic obstructive pulmonary disease (COPD); sarcoidosis;
hypersensitivity pneumonitis (HP).

In a further embodiment, the pulmonary inflammatory disease is asthma

In one embodiment of the invention, the compound for use in the method of the invention is administered orally, parenteraly, topically or by inhalation.

Alternatively, the compound is administered orally, topically, or by inhalation.

In one embodiment of the invention, the compound for use in the method of the invention is administered orally.

In one embodiment, the compounds described herein are useful for the manufacture of a medicament for treating pulmonary inflammatory diseases.

In one embodiment, there are novel compounds provided having the formula: 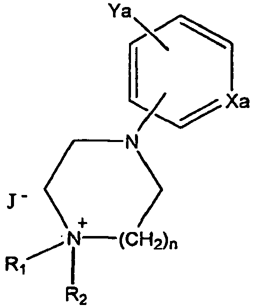 wherein R₁ and R₂ are independently lower alkyl of 1 to 10 carbon atoms,
Xa is CH or N,
Ya is one or more substituent selected from hydrogen, halogen, amino, amidino, amido, azido, cyano, guanido, hydroxyl, nitro, nitroso, urea, sulfate, sulfite, sulfonate, sulphonamide, phosphate, phosphonate, acyl, acyloxy, alkyl of 1 to 6 carbon atoms, alkoxy of 1 to 6 carbon atoms, alkylthio of 1 to 6 carbon atoms, alkylamino of 1 to 6 carbon atoms, alkanol of 1 to 6 carbon atoms, aralkyl, aryl of 6 to 10 carbon atoms and 3 to 10 membered heterocycle
n is 2,
J is a counter ion.

In a further embodiment R₁ and R₂ are independently optionally substituted alkyl of 1 to 6 carbon atoms;
Xa is CH;
Ya is one or more substituent selected from hydrogen, halogen, amino, amido, hydroxyl, alkyl of 1 to 6 carbon atoms, alkoxy of 1 to 6 carbon atoms and alkanol of 1 to 6 carbon atoms;
n is 2;
J is a halogen.

In one embodiment, the compound has the formula: 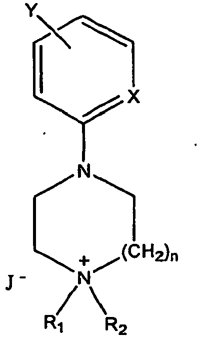 wherein R₁ and R₂ are independently optionally substituted alkyl of 1 to 6 carbon atoms;
X is CH;
Y is one or more substituent selected from hydrogen, halogen, amino, amido, hydroxyl, alkyl of 1 to 6 carbon atoms, alkoxy of 1 to 6 carbon atoms, alkanol of 1 to 6 carbon atoms;
n is 2;
J is a halogen.

In a further embodiment, R₁ and R₂ are independently selected from methyl, ethyl, n-propyl, or i-propyl;
X is CH;
Y is hydrogen;
n is 2;
J is a halogen.

In an alternative embodiment, the compound has the formula: 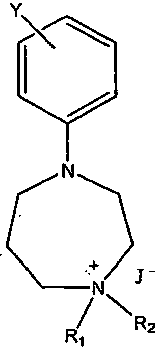 wherein R₁ and R₂ are independently selected from methyl, ethyl, n-propyl, or i-propyl;
Y is hydrogen;
J is a halogen.

In still a further embodiment, the compound has the formula:

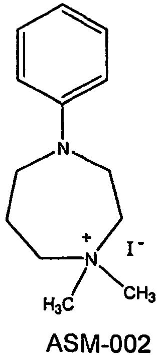

The first nicotinic receptor agonists include dimethylphenylpiperazinium (DMPP) analogues thereof.

Alternatively, nicotinic receptor agonists that can be used for the treatments and uses according to the invention include the following nicotinic receptor agonists and analogues thereof:
DMPP analogs thereof

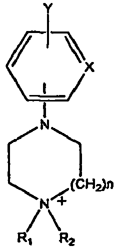

| **Compound** | **R₁** | **R₂** | **X** | **Y** | **N** |
|---|---|---|---|---|---|
| | CH₃ | CH₂CH₂CH₃ | CH | - | 2 |
| | CH₂CH₃ | CH₂CH₃ | CH | - | 2 |
| | CH₂CH₃ | CH₃ | CH | - | 2 |
| | CH₃ | CH₃ | CH | - | 2 |

Of particular interest for the treatment of inflammatory pulmonary diseases, are the following analogues of DMPP, and having the formula: 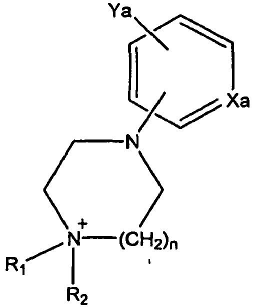 in which R₁ is methyl or ethyl, R₂ is methyl, ethyl or propyl, X is CH, Y is hydrogen, n is 2.

The term "lower alkyl" represents a linear, branched or cyclic hydrocarbon moiety having 1 to 10 carbon atoms and preferably 1 to 6 carbon atoms, which may have one or more unsaturation in the chain, and is optionally substituted. Examples include but are not limited to methyl, ethyl, propyl, isopropyl, butyl, isobutyl, sec-butyl, tert-butyl, pentyl, isopentyl, neopentyl, tert-pentyl, hexyl, isohexyl, neohexyl, allyl, vinyl, acetylenyl, ethylenyl, propenyl, isopropenyl, butenyl, isobutenyl, hexenyl, butadienyl, pentenyl, pentadienyl, hexenyl, hexadienyl, hexatrienyl, heptenyl, heptadienyl, heptatrienyl, octenyl, octadienyl, octatrienyl, octatetraenyl, propynyl, butynyl, pentynyl, hexynyl, cyclopropyl, cyclobutyl, cyclohexenyl, cyclohex-dienyl and cyclohexyl. The term "lower alkyl" is also meant to include alkyls in which one or more hydrogen atom is replaced by a halogen, ie. an alkylhalide. Examples include but are not limited to trifluoromethyl, difluoromethyl, fluoromethyl, trichloromethyl, dichloromethyl, chloromethyl, trifluoroethyl, difluoroethyl, fluoroethyl, trichloroethyl, dichloroethyl, chloroethyl, chlorofluoromethyl, chlorodifluoromethyl, dichlorofluoroethyl.

The term "lower alkoxy" represents an alkyl which is covalently bonded to the adjacent atom through an oxygen atom. Examples include but are not limited to methoxy, ethoxy, propoxy, isopropoxy, butoxy, isobutoxy, sec-butoxy, tert-butoxy, pentyloxy, isopentyloxy, neopentyloxy, tert-pentyloxy, hexyloxy, isohexyloxy and neohexyloxy.

The term "lower Alkylthio "represents an alkyl which is covalently bonded to the adjacent atom through a sulfur atom Examples include but are not limited to methylthio, ethylthio, propylthio, isopropylthio, butylthio, isobutylthio, sec-butylthio and tert-butylthio.

The term "lower Alkylamino" represents an alkyl which is covalently bonded to the adjacent atom through a nitrogen atom and may be monoalkylamino or dialkylamino, wherein the alkyl groups may be the same or different. Examples include but are not limited to methylamino, dimethylamino, ethylamino, diethylamino, methylethylamino, propylamino, isopropylamino, butylamino, isobutylamino, sec-butylamino, tert-butylamino, pentylamino, isopentylamino, neopentylamino, tert-pentylamino, hexylamino, isohexylamino and neohexylamino,

The term "lower alkanol" represents an "alkyl" moiety for which one of the hydrogens has been replaced by an hydroxyl group. The term alkanol is also meant to include alkanol in which one or more hydrogen atoms is replaced by a halogen. Examples include but are not limited to methanol, ethanol, propanol, isopropanol, butanol, ethyleneglycol, propyleneglycol, cyclopropanol or trifluoroethanol or fluoromethanol.

The term "aralkyl" represents an aryl group attached to the adjacent atom by a C₁₋₆ alkyl Examples include but are not limited to benzyl, benzhydryl, trityl, phenethyl, 3-phenylpropyl, 2-phenylpropyl, 4-phenylbutyl and naphthylmethyl.

The term "aryl" represents a carbocyclic moiety containing at least one benzenoid-type ring (i.e. may be monocyclic or polycyclic) having 6 to 10 carbon atoms, and which may be optionally substituted with one or more substituents. Alternatively, the ring may be containing 6 carbon atoms. Examples include but is not limited to phenyl, tolyl, dimethyphenyl, aminophenyl, anilinyl, naphthyl, anthryl, phenanthryl or biphenyl,

The term "Acyl" is defined as a radical derived from a carboxylic acid, obtained by replacement of the -OH group. Like the acid to which it is related, an acyl radical may be straight chain, branched chain or cyclic aliphatic or aromatic. Examples include but are not limited to formyl, acetyl, propionyl, butyryl, isobutyryl, valeryl, isovaleryl, pivaloyl, caproyl, isocaproyl, acryloyl, propioloyl, methacryloyl, crotonoyl, isocrotonoyl, benzoyl, naphthoyl, toluoyl, cinnamoyl, furoyl, glyceroyl, salicyloyl.

The term "Acyloxy" represents an acyl which is covalently bonded to the adjacent atom through an oxygen atom. Examples include but are not limited to formyloxy, acetyloxy, propionyloxy, butyryloxy, isobutyryloxy, valeryloxy, isovaleryloxy, pivaloyloxy, caproyloxy, isocaproyloxy, acryloyloxy, propioloyloxy, methacryloyloxy, crotonoyloxy, isocrotonoyloxy, benzoyloxy, naphthoyloxy, toluoyloxy, hydroatropoyloxy, atropoyloxy, cinnamoyloxy, furoyloxy, glyceroyloxy, tropoyloxy, benziloyloxy, salicyloyloxy, anisoyloxy, vanilloyloxy, veratroyloxy, piperonyloyloxy, protocatechuoyloxy and galloyloxy, with preference given to formyloxy, acetyloxy, propionyloxy, butyryloxy, isobutyryloxy, valeryloxy, isovaleryloxy, pivaloyloxy, benzoyloxy and naphthoyloxy. '

The term "halogen atom" is specifically a fluoride atom, chloride atom, bromide atom or iodide atom.

The term "counterion" is meant to include ion that accompanies an ionic species in order to maintain electric neutrality. Examples of counterion as used herein include but are not limited to fluoride, chloride, bromide, iodide, sulfate, sulfonate.

The term "independently" means that a substituent can be the same or a different definition for each item.

The term "heterocycle" represents a 3 to 10 membered optionally substituted saturated, unsaturated or aromatic cyclic moiety wherein said cyclic moeity is interrupted by at least one heteroatom selected from oxygen (O), sulfur (S) or nitrogen (N). Alternatively, heterocycles may be 3 to 6 membered ring or 5 to 6 membered ring. Heterocycles may be monocyclic or polycyclic rings. Examples include but are not limited to azepinyl, aziridinyl, azetyl, azetidinyl, diazepinyl, dithiadiazinyl, dioxazepinyl, dioxolanyl, dithiazolyl, furanyl, isooxazolyl, isothiazolyl, imidazolyl, morpholinyl, morpholino, oxetanyl, oxadiazolyl, oxiranyl, oxazinyl oxazolyl, piperazinyl, pyrazinyl, pyridazinyl, pyrimidinyl, piperidyl, piperidino, pyridyl, pyranyl, pyrazolyl, pyrrolyl, pyrrolidinyl, thiatriazolyl, tetrazolyl, thiadiazolyl, triazolyl, thiazolyl, thienyl, tetrazinyl, thiadiazinyl, triazinyl, thiazinyl and thiopyranyl, furoisoxazolyl, imidazothiazolyl, thienoisothiazolyl, thienothiazolyl, imidazopyrazolyl, cyclopentapyrazolyl, pyrrolopyrrolyl, thienothienyl, thiadiazolopyrimidinyl, thiazolothiazinyl, thiazolopyrimidinyl, thiazolopyridinyl, oxazolopyrimidinyl, oxazolopyridyl, benzoxazolyl, benzisothiazolyl, benzothiazolyl, imidazopyrazinyl, purinyl, pyrazolopyrimidinyl, imidazopyridinyl, benzimidazolyl, indazolyl, benzoxathiolyl, benzodioxolyl, benzodithiolyl, indolizinyl, indolinyl, isoindolinyl, furopyrimidinyl, furopyridyl, benzofuranyl, isobenzofuranyl, thienopyrimidinyl, thienopyridyl, benzothienyl, cyclopentaoxazinyl, cyclopentafuranyl, benzoxazinyl, benzothiazinyl, quinazolinyl, naphthyridinyl, quinolinyl, isoquinolinyl, benzopyranyl, pyridopyridazinyl and pyridopyrimidinyl.

For the purposes of the present application, the term "animal" is meant to signify human beings, primates, domestic animals (such as horses, cows, pigs, goats, sheep, cats, dogs, guinea pigs, mice, etc.) and other mammals. Generally, this term is used to indicate living creatures having highly developed vascular systems.

For the purposes of the present invention, agonists or agents or ligands are molecules or compounds that bind to and modulate the function of the nicotinic receptor. Preferred agents are receptor-specific and do not cross the blood-brain barrier, such as DMPP. Useful agents may be found within numerous chemical classes, though typically they are organic compounds and preferably, small organic compounds. Small organic compounds have a molecular weight of more than 150 yet less than about 4,500, preferably less than about 1500, more preferably, less than about 500. Exemplary classes include peptides, saccharides, steroids, heterocyclics, polycyclics, substituted aromatic compounds, and the like.

Nicotinic agonists would not necessarily replace all drugs that are currently used to specifically treat inflammatory lung diseases and the airflow obstruction that is often associated with these diseases. Bronchodilators remain useful for the immediate release of bronchospasms. However, bronchodilators have no effect on the underlying cause of inflammation.

Nicotinic agonists may be useful as a steroid sparing or replacing drug. By targeting their delivery to the lung phagocytes, these drugs could be helpful in controlling both airway and interstitial inflammation. One major advantage of nicotinic agonists over corticosteroids, besides being expected to have fewer side effects, is the fact that these agonists may have a direct effect on fibroblasts and could therefore prevent or reverse fibrosis in the airways and in the lungs, something corticosteroids cannot do. Interstitial fibrosis is the hallmark if IPF, a major sequel of HP and sarcoidosis, and airway fibrosis is a prevailing finding in chronic asthma (57).

Other substances are actively being studied as potential new treatments for inflammatory lung diseases. Many cytokines are specifically targeted (e.g. IL-5, IL-13, IL-16 and the like) (62). It is believed that because of the complexity of pathways involved in inflammation, any one specific cytokine or other inflammatory mediator is unlikely to have a significant impact on the treatment of these lung diseases. Nicotinic receptor agonists as well as analogs and derivatives thereof, not unlike corticosteroids, have the advantage of targeting a broad spectrum of the inflammatory response. Therein lies their potential in the treatment of inflammatory lung diseases.

Selected agents may be modified to enhance efficacy, stability, pharmaceutical compatibility, and the like. Structural identification of an agent may be used to identify, generate, or screen additional agents. For example, where peptide agents are identified, they may be modified in a variety of ways as described above, e.g. to enhance their proteolytic stability. Other methods of stabilization may include encapsulation, for example, in liposomes, etc. The subject binding agents are prepared in any convenient way known to those skilled in the art.

For therapeutic uses, agents affecting nicotinic receptor function may be administered by any convenient means. Small organics are preferably administered orally; other compositions and agents are preferably administered parenterally, conveniently in a pharmaceutically or physiologically acceptable carrier, e.g., phosphate buffered saline, or the like. Typically, the compositions are added to a retained physiological fluid such as blood or synovial fluid.

In accordance with the invention, there is provided another embodiment which is a pharmaceutical composition for treating pulmonary inflammatory diseases comprising a nicotinic receptor agonist and a pharmaceutically acceptable excipient.

The carrier(s) or excipient(s) must be "acceptable" in the sense of being compatible with the other ingredients of the formulation and not being deleterious to the recipient thereof.

In an alternative embodiment, there is provided a pharmaceutical composition for treating pulmonary inflammatory diseases comprising
i) a compound of formula: 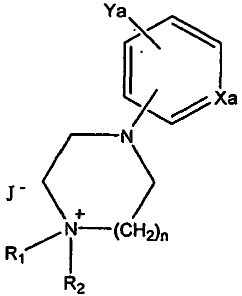
wherein R₁ and R₂ are independently lower alkyl of 1 to 10 carbon atoms,
Xa is CH or N,
Ya is one or more optional substituent selected from halogen, amino, amidino, amido, azido, cyano, guanido, hydroxyl, nitro, nitroso, urea, sulfate, sulfite, sulfonate, sulphonamide, phosphate, phosphonate, acyl, acyloxy, alkyl of 1 to 6 carbon atoms, alkoxy of 1 to 6 carbon atoms, alkylthio of 1 to 6 carbon atoms, alkylamino of 1 to 6 carbon atoms, alkanol of 1 to 6 carbon atoms, aralkyl, aryl of 6 to 10 carbon atoms and 3 to 10 membered heterocycle
n is 2,
J is a counter ion.

In one embodiment, the pharmaceutical composition as defined herein may be further comprising one or more therapeutic agent selected from a bronchodilating agent, an anti-inflammatory agent, a leukotriene receptor antagonist or a phosphodiesterase inhibitor (PDE) such as PDE IV.

In a further embodiment, the bronchodilating agent is β2 agonists or anticholinergics.

In a further embodiment, the an anti-inflammatory agent is corticosteroids.

In a further embodiment, the PDE inhibitor is PDE IV.

In another embodiment, the present invention provides a combination comprising a therapeutically effective amount of a compound useful in the method of the present invention, and a therapeutically effective amount of at least one or more therapeutic agent.

It will be clear to a person of ordinary skill that if a further additional therapeutic agent is required or desired, ratios will be readily adjusted. It will be understood that the scope of combinations described herein is not limited to the therapeutic agents listed herein, but includes in principles any therapeutic agent useful for the prevention and treatment of pulmonary inflammatory diseases.

For peptide agents, the concentration will generally be in the range of about 50 to 500 µg/ml. Alternatively, it may administered in an acceptable range of from about 1 mg to a few 10g or more per Kg in a body weight basis) in the dose administered. Other additives may be included, such as stabilizers, bactericides, etc. These additives will be present in conventional amounts.

It will be appreciated that the amount of a compound of the invention required for use in treatment will vary not only with the particular compound selected but also with the route of administration, the nature of the condition for which treatment is required and the age and condition of the patient and will be ultimately at the discretion of the attendant physician or veterinarian. Generally, the amount administered will be empirically determined, typically in the range of about 10µg to 1000 mg/kg of the recipient or 10µg to 100 mg/kg or 10µg to 1 mg/kg for example.

The desired dose may conveniently be presented in a single dose or as divided dose administered at appropriate intervals, for example as two, three, four or more doses per day.

While it is possible that, for use in therapy, a compound or combination of the invention may be administered as the raw chemical it is preferable to present the active ingredient as a pharmaceutical composition.

As examples, many such therapeutics are amenable to direct injection or infusion, topical, intratracheal/nasal administration e.g. through aerosol, intraocularly, or within/on implants (such as collagen, osmotic pumps, grafts comprising appropriately transformed cells, etc. with therapeutic peptides.

Pharmaceutical compositions also include those suitable for oral, nasal, topical (including buccal and sub-lingual), transdermal, or parenteral (including intramuscular, sub-cutaneous and intravenous) administration or in a form suitable for administration by inhalation. The formulations may, where appropriate, be conveniently presented in discrete dosage units and may be prepared by any of the methods well known in the art of pharmacy. All methods include the step of bringing into association the active compound with liquid carriers or finely divided solid carriers or both and then, if necessary, shaping the product into the desired formulation.

Pharmaceutical compositions suitable for oral administration may conveniently be presented as discrete units such as capsules, cachets or tablets each containing a predetermined amount of the active ingredient; as a powder or granules; as a solution, a suspension or as an emulsion. The active ingredient may also be presented as a bolus, electuary or paste. Tablets and capsules for oral administration may contain conventional excipients such as binding agents, fillers, lubricants, disintegrants, or wetting agents. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), or preservatives.

The compounds and combinations according to the invention may also be formulated for parenteral administration (e.g. by injection, for example bolus injection or continuous infusion) and may be presented in unit dose form in ampoules, prefilled syringes, small volume infusion or in multi-dose containers with an added preservative. The compositions may take such forms as suspensions, solutions, or emulsions in oily or aqueous vehicles, and may contain formulatory agents such as suspending, stabilizing an/or dispersing agents. Alternatively, the active ingredient may be in powder form, obtained by aseptic isolation of sterile solid or by lyophilisation from solution, for constitution with a suitable vehicle, e.g. sterile, pyrogen-free water, before use.

Compositions suitable for topical administration in the mouth include lozenges comprising active ingredient in a flavoured base, usually sucrose and acacia or tragacanth; pastilles comprising the active ingredient in an inert base such as gelatin and glycerin or sucrose and acacia; and mouthwashes comprising the active ingredient in a suitable liquid carrier.

For administration by inhalation the compounds and combinations according to the invention are conveniently delivered from an insufflator, nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount.

Alternatively, for administration by inhalation, the compounds and combinations according to the invention may take the form of a dry powder composition, for example a powder mix of the compound and a suitable powder base such as lactose or starch. The powder composition may be presented in unit dosage form in, for example, capsules or cartridges or e.g. gelatin or blister packs from which the powder may be administered with the aid of an inhalator or insufflator.

Two animal models were used to study the effects of nicotinic antagonists in inflammatory pulmonary diseases: an HP model and an asthma model. With both of these models, the effects of nicotinic receptor agonists (both selective and non-selective) were studied on lung physiology, and inflammation. *In vitro* studies were performed using isolated inflammatory cells from the animal studies or from patients as well as commercially available cell lines in an attempt to understand the mechanisms by which nicotinic agonists down-regulate inflammation.

Initially, experiments were conducted with non-specific agonists, i.e agonists that bind to all nicotinic receptor subunits (nicotine, dimethylphenylpiperazinium (DMPP) and epibatidine) (13, 42). A β4 subunit specific agonist, cytisine (42), was also tested to see whether a specific stimulation could also have anti-inflammatory effects.

### EXAMPLE X

### Effects of DMPP analogs

Based on our DMPP results, four (4) new DMPP analogs were developed and tested for their anti-inflammatory effects, improved hyper-responsiveness properties and smooth muscle-relaxing effects. Similarly to DMPP, ASM-002, ASM-003, ASM-004 and ASM-005 are synthetic agonists of nicotinic acethylcholine receptors. They are highly hydrophilic due to their quaternary salt structure, and therefore are not likely to cross easily the blood-brain barrier. Consequently they are less likely to induce addiction.

### EXAMPLE XI

### Anti- inflammatory effects:

### Effect of DMPP analogs on Tumor necrosis factor (TNF) release

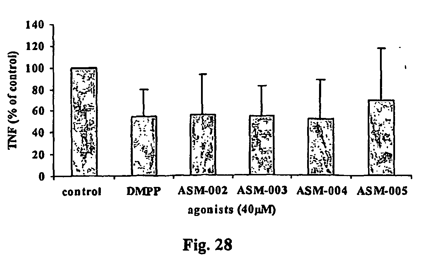

Human monocytes were isolated from the blood of asthmatic patients by Ficoll-paque density gradient, let to adhere to tissue culture plates and stimulated with LPS (100ng/ml) for 18 hours at 37°C with or without increasing concentrations of nicotine. The release of TNF, a potent pro-inflammatory mediator, was measured in the cell culture supernatant by the ELISA method. Results are expressed as a percentage release from LPS-stimulated untreated cells (Fig. 28). Except for ASM-005, all analogs tested had an inhibitory effect on TNF release (n=8 to 10; p from 0.01 to 0.007).

### EXAMPLE XII

### Effect of DMPP analogs on Leukotriene C4 (LTC4)production.

Blood eosinophils, the most increased inflammatory cells in asthma, were isolated by negative selection using bead-conjugated anti-CD16 monoclonal antibody and magnetic activating cell sorting. Cells were pre-incubated for 18hours with the various DMPP analogs and then stimulated with 1 µM platelet-activating factor (PAF) to produce LTC4 which was measured by enzyme immunoassay.
The results indicate that 3 out of 4 analogs tested are able to down-regulate LTC4 release (Table 1).

**Table 1 Effects of DMPP and analogs on LTC4 release.**

| | LTC4 pg/ml |
|---|---|
| - | 1725.80 |
| DMPP | 545.00 |
| ASM002 | 246.40 |
| ASM003 | 613.90 |
| ASM004 | 601.60 |

### EXAMPLE XIII

### Smooth muscle relaxing effects:

### Effect of DMPP analogs on mouse tracheal airway smooth muscle responsiveness.

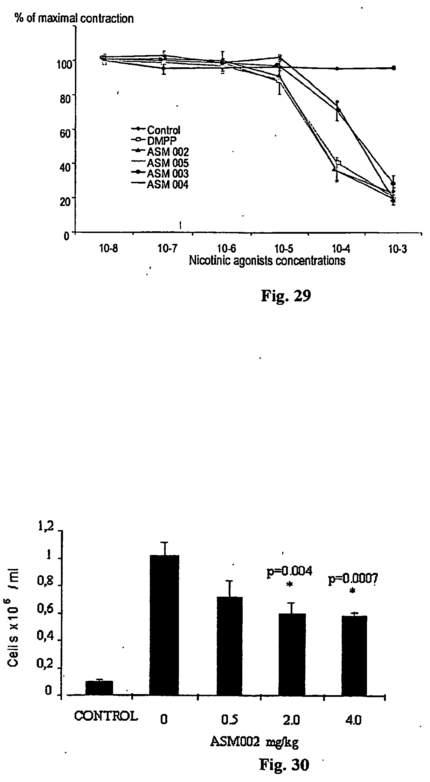

To demonstrate the relaxing effects of DMPP analogs on airway smooth muscle cells, isometric studies were performed on isolated mouse tracheas. Tracheal rings were mounted isometrically to force transducers in organ baths containing Krebs bicarbonate solution at 37°C and bubbled with 95% O₂- 5% CO₂, pre-contracted with submaximal concentration of metacholine (10⁻⁵) and cumulative doses of the analogs were added to the baths. Changes of tension are recorded. Results are expressed as a percentage of maximal contraction (Fig. 29).
Similarly to DMPP, its analogs induced a dose dependant relaxation of tracheal smooth muscles pre-contracted with metacholine.

Overall these results indicated that ASM-002, ASM-003, ASM-004 and ASM-005 the new synthesized analogs presented similar anti-inflammatory and smooth-muscle relaxing effects as DMPP.

### EXAMPLE XIV

### Mouse model

### Effects of ASM-002 on lung inflammation

Ovalbumin-sensitized mice (ri=8) were challenged with the allergen and simultaneously treated intra-nasally with increasing concentrations of ASM-002 (0.5 to 4 mg/kg/d) for 3 days. The number of cells recovered by broncho-alveolar lavage was used as a measure of lung inflammation.

As shown in Fig. 30, ASM-002 significantly inhibits in a dose dependant manner the cellular inflammation in the lungs of asthmatic mice (ED₅₀ = 0.71 mg/kg, n = 8).

### EXAMPLE XV

### Mouse model

### Effects of ASM-002 on lung resistance in a mouse model of asthma

Lung response to a broncho-constrictive agent, metacholine, was measured by a Flexi-vent® apparatus. Ovalbumin-sensitized animals were treated intra-nasally with ASM-002 (4mg/kg) during 3 days and 10 minutes prior to the metacholine challenge and compared to untreated OVA-sensitized animals. A negative control group of un-sensitized animals and a positive control group that received Salbutamol (Ventolin) 10 minutes before the metacholine challenge were also included.

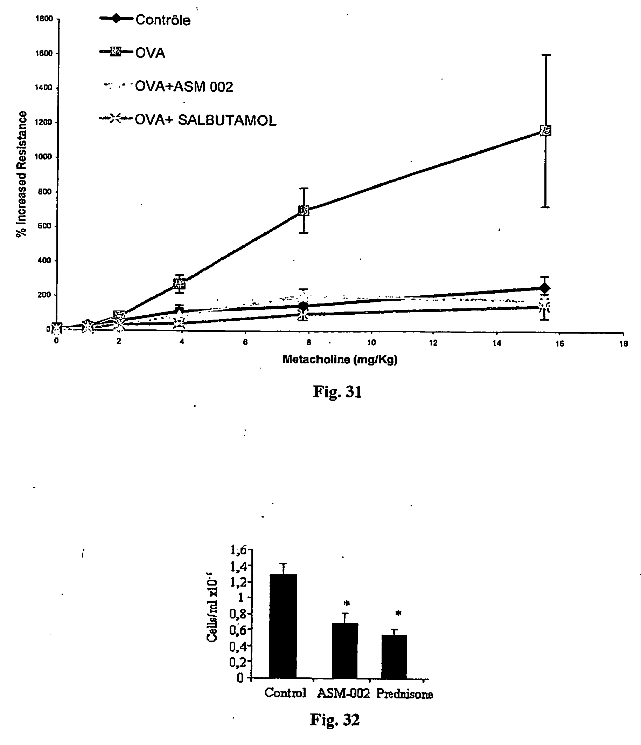

The results show (Fig. 31) an increase in lung resistance, induced by metacholine, in OVA-sensitized mice compared to the negative control group. A significant reduction (return to baseline levels) in lung resistance is observed in ASM-002-treated mice compared to untreated mice (n=8, p<0.02). This effect is similar to that obtained with Salbutamol (Ventolin™), the most common brochodilator currently used in asthma to relieve broncho-constriction symptoms (n=4, p<0.02).

### EXAMPLE XVI

### Dog asthma model

In this model 12 dogs naturally sensitized to the roundworm *Ascaris suum* were used in a cross-over study design. Four groups of 3 dogs were randomly formed, exposed to the allergen, and each animal was left either untreated or received alternalively, ASM-002 (4mg/kg 2x day in the food), or prednisone (1mg/kg 1x day in the food), the most commonly used corticosteroid drug used to treat inflammation in asthma.

### Comparative effects of ASM-002 and Prednisone TM on lung inflammation

Cellular inflammation was evaluated in the bronchoalveolar lavages.

As shown in Fig. 32, ASM-002 (8 mg/kg) inhibits significantly the cellular inflammation in the lungs of asthmatic dogs with a similar efficacy as Prednisone™, the most frequently used anti-inflammatory drug (n = 12, p< 0.05)..

### EXAMPLE XVII

### Effects of ASM-002 in a dog model of lung hyper-responsiveness.

Hyper-responsiveness is described as the capacity of the lung to react (to increase lung resistance) to a non-specific external stimuli like metacholine or to allergens. A hyper-responsive allergen-sensitized dog (asthmatic) will react to lower concentrations of metacholine compared to a non allergic dog. Similarly, improvement in lung hyper-responsivenes is shown by an increase in metacholine concentrations necessary to induce the same level of lung resistance.

Increasing concentrations of metacholine were administered with or without treatment with ASM-002 or Prednisone™ and lung resistance recorded.

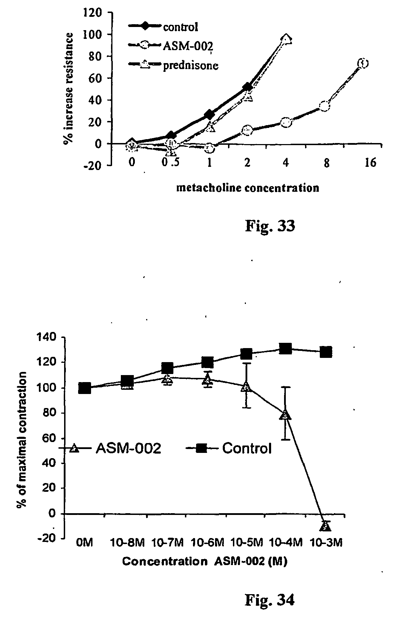

As shown in Fig. 33, ASM-002 decreased lung resistance in 7 out of 12 hyperresponsive dogs. None of the 12 dogs showed improved hyper-responsiveness with prednisone (p=0.005).

### EXAMPLE XVIII

### Muscle-relaxing properties of ASM-002 .

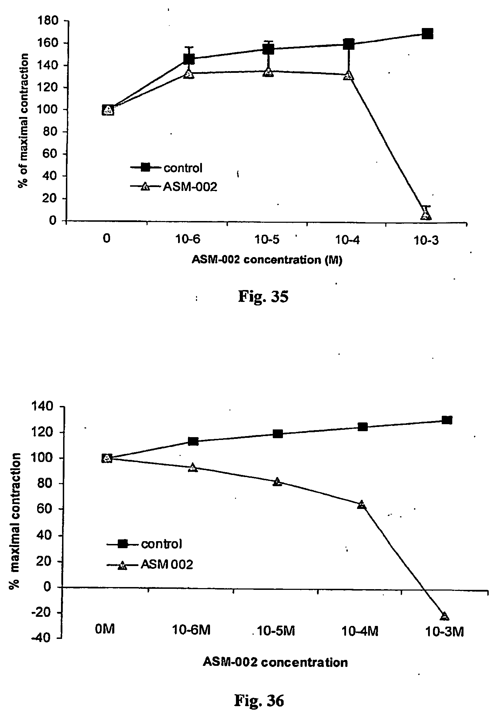

To further demonstrate the relaxing effects of ASM-002 on airway smooth muscle cells, isometric studies were performed on isolated mouse tracheas, bronchial rings from dog's lungs and bronchial rings from resected human lungs. As described previously, tracheal or bronchial rings were mounted isometrically to force transducers in organ baths containing Krebs bicarbonate solution at 37°C and bubbled with 95% O₂- 5% CO₂,pre-contracted with submaximal concentration of metacholine cumulative doses of ASM-002 added. Changes of tension are recorded. Results are expressed as a percentage of maximal contraction for mouse (Fig. 34, p=0.002), dog (Fig. 35, p=0.004) and human (Fig. 36)
These results of examples XIV to XVIII showed that ASM-002 present a relaxing effect on pre-contracted mice tracheas, dog and human bronchi.

### EXAMPLE XX

### In vitro studies.

The anti-inflammatory activity of ASM-002 was observed *in vivo* in mice and dogs in previous Examples. To further characterize this effect, the drug was tested for its capacity to inhibit the release of 2 potent inflammatory mediators by human blood cells isolated from asthmatic patients

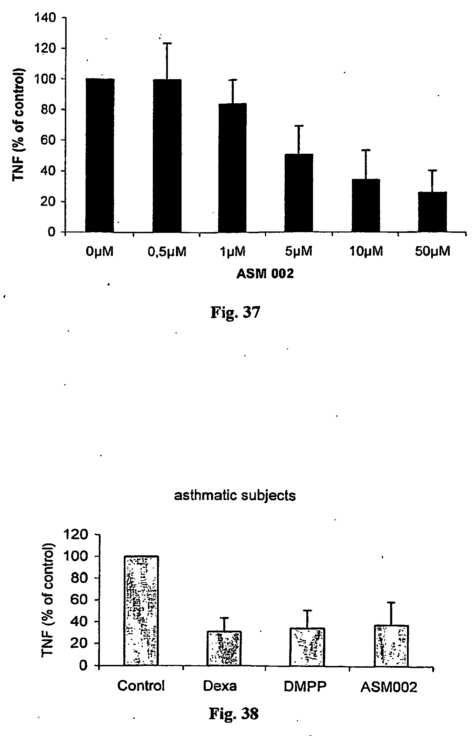

Tumor necrosis factor (TNF) is a mediator released in inflammatory states. Human blood monocytes were stimulated *in vitro* with lipopolysaccharide (LPS) to produce large amounts of TNF, increasing doses of ASM-002 were added and the levels of TNF were measured (Fig. 37, EC₅₀= 3µM, n=6, p= 0,0045 at 5 µm, 0,0014 at 10 µm and 0,0003 at 50 µm). A dose-dependant inhibition of TNF release was observed with ASM-002

### EXAMPLE XXI

### Comparative effects of ASM-002 with DMPP and dexamethasone on TNF production by LPS-stimulated blood monocytes.

As shown in Fig. 38, results are expressed as a percentage from untreated control cells, all drugs were added at a 40 µM concentration and are the mean of 5 different experiments (5 subjects). ASM-002 inhibits TNF release from human blood monocytes as well as dexamethasone and DMPP (p= from 0.02 to 0.001)

Leukotriene C₄ (LTC₄) is a inflammatory lipid mediator highly produced in asthma, it is released in large amounts by blood eosinophils.

Human blood eosinophils were isolated from blood of asthmatic patients, stimulated *in vitro* with platelet activating factor (PAF) to produce large amounts of LTC4 , and treated or not with 80 µM ASM-002.

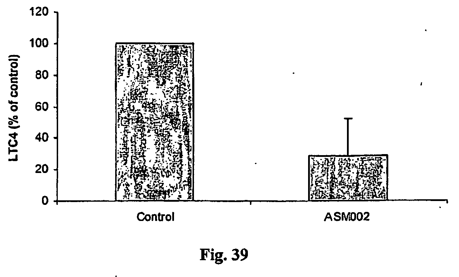

A significant inhibition of LTC4 production by ASM-treated eosinophils was observed (Fig. 39, p=0.0007). The results represent an average of 6 different experiments (6 patients).

The results showed that ASM-002 present combined anti-inflammatory and broncho-dilating properties and improved hyperresponsiveness , which could be highly effective for the relief and treatment of asthma and other obstructive respiratory diseases.

### Reference EXAMPLE XXIII

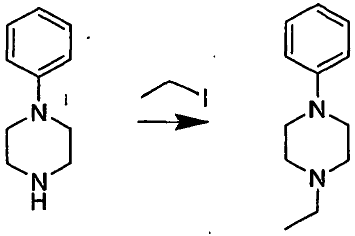

1-Phénylpiperazine (1eq), iodoethane (1 eq), and sodium carbonate (2 eq) were mixed in tert-butanol: The mixture was refluxed for 20 hours. The mixture is then dissolved in chloroform and extracted with water three times. The organic layer was washed with 1 N aqueous HCl solution three times. The aqueous layer was then basified to a basic pH with NaOH pellets. The basic aqueous layer was then extracted with chloroform three times and the combined organic extracts dried over Na₂SO₄ and evaporated to dryness. The crude product was purified using silica gel flash chromatography using a gradient of 0-5% MeOH in chloroform. The desired product was obtained as a yellow oil. (yield. 52%).

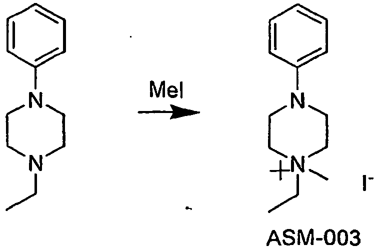

N-ethylphenylpipérazine (1 eq, 0.6 mmol) and iodomethane (excess >10 eq, 1 ml) were stirred in ether at room temperature for 4 days. The resulting white precipitate of ASM-003 was isolate by vacuum filtration. (yield 75%).

### Reference EXAMPLE XXIV

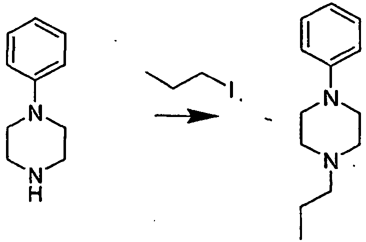

1-Phénylpiperazine (1 eq), iodopropane (1 eq), and sodium carbonate (2 eq) were mixed in tert-butanol. The mixture was refluxed for 20 hours. The mixture is then dissolved in chloroform and extracted with water three times. The organic layer was washed with 1 N aqueous HCl solution three times. The aqueous layer was then basified to a basic pH with NaOH pellets. The basic aqueous layer was then extracted with chloroform three times and the combined organic extracts dried and evaporated to dryness. The crude product was purified using silica gel flash chromatography using a gradient of 0-5% MeOH in chloroform. The desired product was obtained as a yellow oil. (yield. 83%).

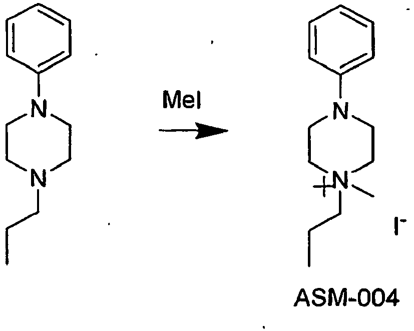

N-propylphenylpiperazine (1 eq, 0.6mmol) and iodomethane (excess >10 eq, 1 ml) were mixed and stirred at room temperature in ether for 2 days. The mixture was then refluxed for 48 hours with an additional amount of iodomethane (>10 eq) with a (1:1) mixture of THF and ether. The mixture was evaporated and diluted in ether to yield a white precipitate of ASM-004 isolated by vacuum filtration. (yield 86%).

### Reference EXAMPLE XXV

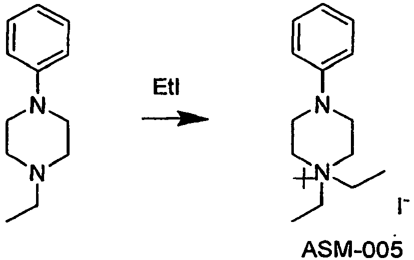

N-ethylphenylpiperazine prepared in example XXIII (1eq, 0.5 mmol) and iodoethane (excess >10 eq, 1 ml) were stirred in ether at room temperature for 2 days. The mixture was then refluxed for 48 hours with an additional amount of iodoethane (>10 eq), with a (1:1) mixture of THF and ether. The mixture was evaporated and diluted in ether to yield a white precipitate of ASM-005 isolated by vacuum filtration (yield 62%).
or
N-ethylphenylpiperazine (1 eq, 3.94 mmol) and iodoethane (excess >10 eq, 3 ml) were stirred in acetonitrile at room temperature The mixture was evaporated and diluted in ether to yield a white precipitate of ASM-005 isolated by vacuum filtration (yield 27%).

### Reference EXAMPLE XXVI

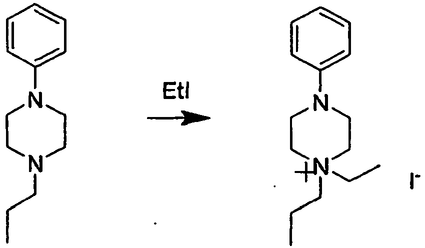

N-propylphenylpiperazine (1 eq, 0.51 mmol) and iodoethane (excess >10 eq, 1 ml) were stirred in ether at room temperature for 2 days The mixture was then refluxed for 48 hours with an additional amount of iodoethane (>10 eq), with a (1:1) mixture of THF and ether. The mixture was evaporated and diluted in ether to yield a white precipitate isolated by vacuum filtration (yield 11%).
or
N-propylphénylpipérazine (1 eq, 0.1 mmol) et l'iodoethane (excess>10 eq, 1 ml) were stirred in refluxing acetone for 24 hours. The mixture was evaporated and diluted in ether to yield a white precipitate isolated by vacuum filtration (yield 75%).

### Reference EXAMPLE XXVII

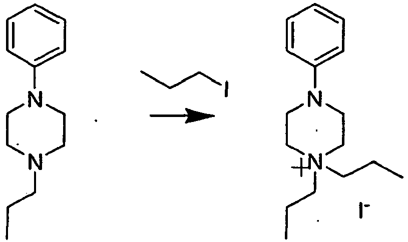

N-propylphenylpiperazine (1 eq, 0.53mmol) and iodopropane (excess >10 eq, 1ml) were stirred in ether at room temperature for 2 days The mixture was then refluxed for 48 hours with an additional amount of iodopropane (>10 eq, 1ml), with a (1:1) mixture of THF and ether. The mixture was evaporated and diluted in ether to yield a white precipitate isolated by vacuum filtration (yield 10%).

### EXAMPLE XXVIII

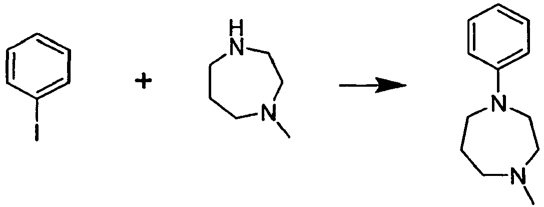

In a flame-dried round bottom flask under nitrogen, iodobenzene (1 eq, 1.47 mmol), N-methylhomopiperazine (1.2 eq, 1.76mmol), ethylene glycol (2 eq, 2.94 mmol), Cul (5% mol) and K3PO4 (2 eq, 2.94 mmol) were suspended in isopropanol (3 ml). The mixture was refluxed with stirring for 17 hours. The resulting mixture was cooled down to room temperature and water was added (5 ml). The mixture was extracted with ether(4 x 10 ml) and the combined organic extracts washed with brine, dried over Na2SO4 and evaporated to dryness under vacuum. The crude product was purified using silica gel flash chromatography using a gradient of 0% a 7.5 % (2M NH3)MeOH in chloroform. The desired product was obtained as a yellow oil. (yield 64%).

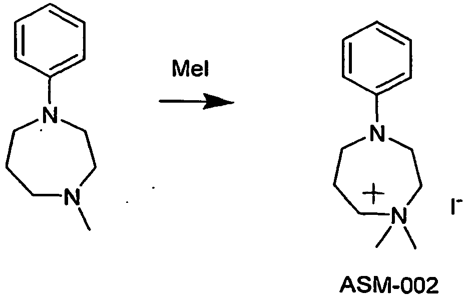

N-methylphenylhomopiperazine (1 eq, 0.36 mmol) and iodomethane (excess >10 eq, 1 ml) were stirred in ether at room temperature for 25 hours. The mixture was evaporated under vacuum, diluted with ether and the resulting white solid filtered under vacuum. 1,1-dimethyl-4-phenylhomopiperazinium iodide (Yield: 66%). Melting point : 158-160.
¹H NMR DMSO-d6 (ppm): (q, 2H) 7.18, (q, 2H) 6.74, (t, 1H) 6.64, (br s, 2H, 3.74), (m, 2H) 3.52, (m, 2H) 3.44, (t, 2H) 3.40, (s, 6H) 3.17, (bs s, 2H) 2.21.
¹³C NMR DMSO-d6: 149, 129, 117, 112, 66, 65, 53, 47, 43, 22.

### REFERENCES

1. Cormier, Y., J. Belanger, and P. Durand. 1985. Factors influencing the development of serum precipitins to farmer's lung antigen in Quebec dairy farmers. Thorax 40(2):138-42.
2. Cormier, Y., L. Gagnon, F. Berube-Genest, and M. Fournier. 1988. Sequential bronchoalveolar lavage in experimental extrinsic allergic alveolitis. The influence of cigarette smoking. Am Rev Respir Dis 137(5):1104-9.
3. Cormier, Y., E. Israel-Assayag, G. Bedard, and C. Duchaine. 1998. Hypersensitivity pneumonitis in peat moss processing plant workers. Am J Respir Crit Care Med 158(2):412-7.
4. Gariepy, L., Y. Cormier, M. Laviolette, and A. Tardif. 1989. redictive value of bronchoalveolar lavage cells and serum precipitins in asymptomatic dairy farmers. Am Rev Respir Dis 140(5):1386-9.
5. Lawrence, E. C., T. B. Fox, R. B. Teague, K. Bloom, and R. K. Wilson. 1986. Cigarette smoking and bronchoalveolar T cell populations in sarcoidosis. Ann N Y Acad Sci 465:657-64.
6. Valeyre, D., P. Soler, C. Clerici, J. Pre, J. P. Battesti, R. Georges, and A. J. Hance. 1988. Smoking and pulmonary sarcoidosis: effect of cigarette smoking on prevalence, clinical manifestations, alveolitis, and evolution of the disease. Thorax 43(7):516-24.
7. Rubin, D. T., and S. B. Hanauer. 2000. Smoking and inflammatory bowel disease. Eur J Gastroenterol Hepatol 12(8):855-62.
8. Thomas, G. A., J. Rhodes, J. T. Green, and C. Richardson. 2000. Role of smoking in inflammatory bowel disease: implications for therapy. Postgrad Med J 76(895):273-9.
9. Guslandi, M. 1999. Nicotine treatment for ulcerative colitis. Br J Clin Pharmacol 48(4):481-4.
10. Guslandi, M. 1999. Long-term effects of a single course of nicotine treatment in acute ulcerative colitis: remission maintenance in a 12-month follow-up study. Int J Colorectal Dis 14(4-5):261-2.
11. Rezvani, A. H., and E. D. Levin. 2001. Cognitive effects of nicotine. Biol Psychiatry 49(3):258-67.
12. Kelton, M. C., H. J. Kahn, C. L. Conrath, and P. A. Newhouse. 2000. The effects of nicotine on Parkinson's disease. Brain Cogn 43(1-3):274-82.
13. Bertram, K.G.1998.Basic and clinical pharmacology.Editions Appelton and Lange. Stanford, Connecticut.
14. Sekhon, H. S., Y. Jia, R. Raab, A. Kuryatov, J. F. Pankow, J. A. Whitsett, J. Lindstrom, and E. R. Spindel. 1999. Prenatal nicotine increases pulmonary alpha7 nicotinic receptor expression and alters fetal lung development in monkeys. J Clin Invest 103(5):637-47.
15. Maus, A. D., E. F. Pereira, P. I. Karachunski, R. M. Horton, D. Navaneetham, K. Macklin, W. S. Cortes, E. X. Albuquerque, and B. M. Conti-Fine. 1998. Human and rodent bronchial epithelial cells express functional nicotinic acetylcholine receptors. Mol Pharmacol 54(5):779-88.
16. Shriver, S. P., H. A. Bourdeau, C. T. Gubish, D. L. Tirpak, A. L. Davis, J. D. Luketich, and J. M. Siegfried. 2000. Sex-specific expression of gastrin-releasing peptide receptor: relationship to smoking history and risk of lung cancer. J Natl Cancer Inst 92(1):24-33.
17. Ferguson, D. G., M. A. Haxhiu, A. J. To, B. Erokwu, and I. A. Dreshaj. 2000. The alpha3 subtype of the nicotinic acetylcholine receptor is expressed in airway-related neurons of the nucleus tractus solitarius, but is not essential for reflex bronchoconstriction in ferrets. Neurosci Lett 287(2):141-5.
18. Singh, S. P., R. Kalra, P. Puttfarcken, A. Kozak, J. Tesfaigzi, and M. L. Sopori. 2000. Acute and chronic nicotine exposures modulate the immune system through different pathways. Toxicol Appl Pharmacol 164(1):65-72.
19. Kalra, R., S. P. Singh, S. M. Savage, G. L. Finch, and M. L. Sopori. 2000. Effects of cigarette smoke on immune response: chronic exposure to cigarette smoke impairs antigen-mediated signaling in T cells and depletes IP3-sensitive Ca(2+) stores. J Pharmacol Exp Ther 293(1):166-71.
20. Sugano, N., K. Shimada, K. Ito, and S. Murai. 1998. Nicotine inhibits the production of inflammatory mediators in U937 cells through modulation of nuclear factor-kappaB activation. Biochem Biophys Res Commun 252(1):25-8.
21. Yates, S. L., M. Bencherif, E. N. Fluhler, and P. M. Lippiello. 1995. Up-regulation of nicotinic acetylcholine receptors following chronic exposure of rats to mainstream cigarette smoke or alpha 4 beta 2 receptors to nicotine. Biochem Pharmacol 50(12):2001-8.
22. Sopori, M. L., and W. Kozak. 1998. Immunomodulatory effects of cigarette smoke. J Neuroimmunol 83(1-2):148-56.
23. Lahmouzi, J., F. Simain-Sato, M. P. Defresne, M. C. De Pauw, E. Heinen, T. Grisar, J. J. Legros, and R. Legrand. 2000. Effect of nicotine on rat gingival fibroblasts in vitro. Connect Tissue Res 41(1):69-80.
24. Geng, Y., S. M. Savage, S. Razanai-Boroujerdi, and M. L. Sopori. 1996. Effects of nicotine on the immune response. II. Chronic nicotine treatment induces T cell anergy. J Immunol 156(7):2384-90.
25. McCrea, K. A., J. E. Ensor, K. Nall, E. R. Bleecker, and J. D. Hasday. 1994. Altered cytokine regulation in the lungs of cigarette smokers. Am J Respir Crit Care Med 150(3):696-703.
26. Ohta, T., N. Yamashita, M. Maruyama, E. Sugiyama, and M. Kobayashi. 1998. Cigarette smoking decreases interleukin-8 secretion by human alveolar macrophages. Respir Med 92(7):922-7.
27. Suzuki, N., S. Wakisaka, Y. Takeba, S. Mihara, and T. Sakane. 1999. Effects of cigarette smoking on Fas/Fas ligand expression of human lymphocytes. Cell Immunol 192(1):48-53.
28. Zia, S., A. Ndoye, V. T. Nguyen, and S. A. Grando. 1997. Nicotine enhances expression of the alpha 3, alpha 4, alpha 5, and alpha 7 nicotinic receptors modulating calcium metabolism and regulating adhesion and motility of respiratory epithelial cells. Res Commun Mol Pathol Pharmacol 97(3):243-62.
29. Zhang, S., and T. M. Petro. 1996. The effect of nicotine on murine CD4 T cell responses. Int J Immunopharmacol 18(8-9):467-78.
30. Bugeon, L., and M. J. Dallman. 2000. Costimulation ofT cells. Am J Respir Crit Care Med 162(4 Pt 2):S164-8.
31. Green, J. M. 2000. The B7/CD28/CTLA4 T-cell activation pathway. Implications for inflammatory lung disease. Am J Respir Cell Mol Biol 22(3):261-4.
32. Lenschow, D. J., T. L. Walunas, and J. A. Bluestone. 1996. CD28/B7 system ofT cell costimulation. Annu Rev Immunol 14:233-58.
33. Walunas, T. L., and J. A. Bluestone. 1998. CTLA-4 regulates tolerance induction and T cell differentiation in vivo. J Immunol 160(8):3855-60.
34. Walunas, T. L., D. J. Lenschow, C. Y. Bakker, P. S. Linsley, G. J. Freeman, J. M. Green, C. B. Thompson, and J. A. Bluestone. 1994. CTLA-4 can function as a negative regulator of T cell activation. Immunity 1(5):405-13.
35. Israel-Assayag, E., A. Dakhama, S. Lavigne, M. Laviolette, and Y. Cormier. 1999. Expression of costimulatory molecules on alveolar macrophages in hypersensitivity pneumonitis. Am J Respir Crit Care Med 159(6):1830-4.
36. Israel-Assayag, E., M. Fournier, and Y. Cormier. 1999. Blockade of T cell costimulation by CTLA4-Ig inhibits lung inflammation in murine hypersensitivity pneumonitis. J Immunol 163(12):6794-9.
37. Larche, M., S. J. Till, B. M. Haselden, J. North, J. Barkans, C. J. Corrigan, A. B. Kay, and D. S. Robinson. 1998. Costimulation through CD86 is involved in airway antigenpresenting cell and T cell responses to allergen in atopic asthmatics. J Immunol 161(11):6375-82.
38. Mathur, M., K. Herrmann, Y. Qin, F. Gulmen, X. Li, R. Krimins, J. Weinstock, D. Elliott, J. A. Bluestone, and P. Padrid. 1999. CD28 interactions with either CD80 or CD86 are sufficient to induce allergic airway inflammation in mice. Am J Respir Cell Mol Biol 21(4):498-509.
39. Nicod, L. P., and P. Isler. 1997. Alveolar macrophages in sarcoidosis coexpress high levels of CD86 (B7.2), CD40, and CD30L. Am J Respir Cell Mol Biol 17(1):91-6.
40. Kesingland, A. C., C. T. Gentry, M. S. Panesar, M. A. Bowes, J. M. Vernier, R. Cube, K. Walker, and L. Urban. 2000. Analgesic profile of the nicotinic acetylcholine receptor agonists, (+)- epibatidine and ABT-594 in models of persistent inflammatory and neuropathic pain. Pain 86(1-2):113-8.
41. Mellon, R. D., and B. M. Bayer. 1999. The effects of morphine, nicotine and epibatidine on lymphocyte activity and hypothalamic-pituitary-adrenal axis responses. J Pharmacol Exp Ther 288(2):635-42.
42. Yokotani, K., M. Wang, S. Okada, Y. Murakami, and M. Hirata. 2000. Characterization of nicotinic acetylcholine receptor-mediated noradrenaline release from the isolated rat stomach. Eur J Pharmacol 402(3):223-9.
43. Yost, C. S., and B. D. Winegar. 1997. Potency of agonists and competitive antagonists on adult- and fetal- type nicotinic acetylcholine receptors. Cell Mol Neurobiol 17(1):35-50.
44. Fecho, K., K. A. Maslonek, L. A. Dykstra, and D. T. Lysle. 1993. Alterations of immune status induced by the sympathetic nervous system: immunomodulatory effects of DMPP alone and in combination with morphine. Brain Behav Immun 7(3):253-70.
45. Thompson, D. C., R. J. Altiere, and L. Diamond. 1990. Nicotinic agonist modulation of feline bronchomotor tone. Clin Exp Pharmacol Physiol 17(2):83-97.
46. Barnes PJ. 2001. Future Advances in COPD Therapy. Respiration 68(5):441-8.
47. Lasky JA and Ortiz, LA. 2001. Antifibrotic therapy for the treatment of pulmonary fibrosis. Am J Med Sci 322(4):213-21.
48. Baron, J. A. 1996. Beneficial effects of nicotine and cigarette smoking: the real, the possible and the spurious. Br Med Bull 52(1):58-73.
49. Waldum, H. L., O. G. Nilsen, T. Nilsen, H. Rorvik, V. Syversen, A. K. Sanvik, O. A. Haugen, S. H. Torp, and E. Brenna. 1996. Long-term effects of inhaled nicotine. Life Sci 58(16):1339-46.
57. Boulet, L. P., H. Turcotte, M. Laviolette, F. Naud, M. C. Bernier, S. Martel, and J. Chakir. 2000. Airway hyperresponsiveness, inflammation, and subepithelial collagen deposition in recently diagnosed versus long-standing mild asthma. Influence of inhaled corticosteroids. Am J Respir Crit Care Med 162(4 Pt 1):1308-13.
58. Dempsey, O. J. 2000. Leukotriene receptor antagonist therapy. Postgrad Med J 76(902):767-73.
59. Busse, W. W. 1998. Leukotrienes and inflammation. Am J Respir Crit Care Med 157(6 Pt 2):S210-3; discussion S247-8.
60. Zisman, D. A., J. P. Lynch, G. B. Toews, E. A. Kazerooni, A. Flint, and F. J. Martinez. 2000. Cyclophosphamide in the treatment of idiopathic pulmonary fibrosis: a prospective study in patients who failed to respond to corticosteroids. Chest 117(6):1619-26.
61. Redington, A. E. 2000. Fibrosis and airway remodelling. Clin Exp Allergy 30 Suppl 1:42-5.
62. Frew, A.J., and Plummeridge MJ. 2001. Alternative agents in asthma. J Allergy Clin Immunol 108(1):3-10.

## Claims

1. A compound of formula: 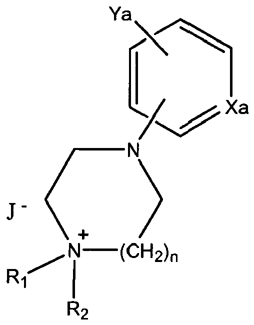 wherein R₁ and R₂ are independently alkyl of 1 to 10 carbon atoms,
Xa is CH or N,
Ya is one or more substituent selected from hydrogen, halogen, amidino, amido, azido, cyano, guanido, hydroxyl, nitroso, urea, sulfate, sulfite, sulfonate, sulphonamide, phosphate, phosphonate, acyl, acyloxy, alkyl of 1 to 10 carbon atoms, alkoxy of 1 to 10 carbon atoms, alkylthio of 1 to 10 carbon atoms, alkylamino of 1 to 10 carbon atoms, alkanol of 1 to 10 carbon atoms, aralkyl, aryl of 6 to 10 carbon atoms and 3 to 10 membered heterocycle
n is 2,
J is a counter ion

2. The compound according to claim 1, wherein R₁ and R₂ are independently selected from methyl, ethyl, n-propyl, or i-propyl;
Xa is CH;
Ya is hydrogen;
n is 2;
J is a halogen.

3. The compound according to claim 1, having the formula:

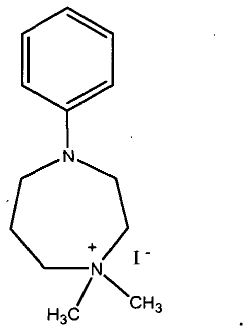

4. The compound according to claim 1, wherein R₁ and R₂ are CH₂CH₃, Xa is CH, Ya is H and n is 2.

5. The compound according to claim 1, wherein said compound has the formula:

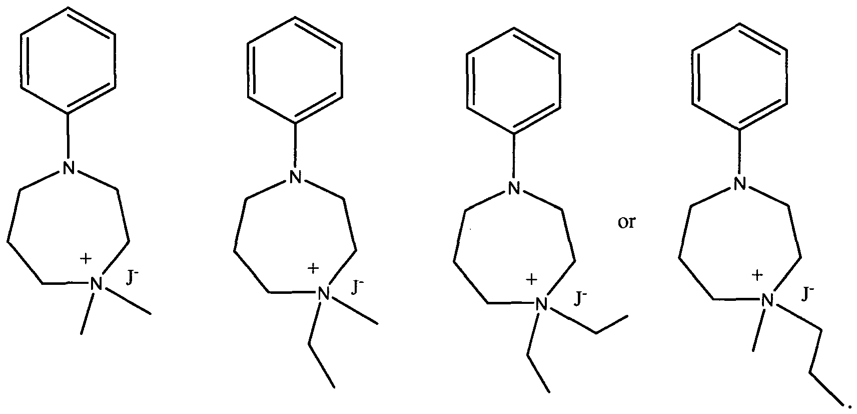

6. The compound according to any one of claims 1 to 5, wherein J is fluoride, chloride, bromide, iodide, sulfate or sulfonate.

7. A pharmaceutical composition for treating pulmonary inflammatory diseases comprising a compound according to any one of claims 1-6, and a pharmaceutically acceptable excipient.

8. The pharmaceutical composition according to claim 7, wherein the pulmonary inflammatory diseases are selected from the group consisting of asthma, chronic obstructive pulmonary disease (COPD), interstitial pulmonary fibrosis (IPF), sarcoidosis, hypersensitivity pneumonitis (HP), chronic HP and bronchiolitis obliterans with organizing pneumonitis (BOOP).

9. The pharmaceutical composition according to claim 7 or 8, further comprising one or more therapeutic agent selected from a bronchodilator, anti-inflammatory therapy, a leukotriene receptor antagonist and phosphodiesterase inhibitors.

10. The pharmaceutical composition according to any one of claims 7 to 9, wherein said pulmonary inflammatory diseases is asthma.

11. A compound for use in treating or preventing pulmonary inflammatory diseases selected from the group consisting of asthma, chronic obstructive pulmonary disease (COPD), interstitial pulmonary fibrosis (IPF), sarcoidosis, hypersensitivity pneumonitis (HP), chronic HP and bronchiolitis obliterans with organizing pneumonitis (BOOP), wherein said compound has the formula: 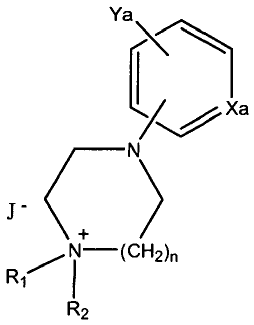 wherein R₁ and R₂ are independently alkyl of 1 to 10 carbon atoms,
Xa is CH or N,
Ya is one or more substituent selected from hydrogen, halogen, amino, amidino, amido, azido, cyano, guanido, hydroxyl, nitro, nitroso, urea, sulfate, sulfite, sulfonate, sulphonamide, phosphate, phosphonate, acyl, acyloxy, alkyl of 1 to 10 carbon atoms, alkoxy of 1 to 10 carbon atoms, alkylthio of 1 to 10 carbon atoms, alkylamino of 1 to 10 carbon atoms, alkanol of 1 to 10 carbon atoms, aralkyl, aryl of 6 to 10 carbon atoms and 3 to 10 membered heterocycle
n is 2,
J is a counter ion.

12. The compound for use according to claim 23, wherein R₁ and R₂ are independently alkyl of 1 to 10 carbon atoms,
Xa is CH or N,
Ya is one or more substituent selected from hydrogen, halogen, amino, amidino, amido, azido, cyano, guanido, hydroxyl, nitro, nitroso, urea, sulfate, sulfite, sulfonate, sulphonamide, phosphate, phosphonate, acyl, acyloxy, alkyl of 1 to 6 carbon atoms, alkoxy of 1 to 6 carbon atoms, alkylthio of 1 to 6 carbon atoms, alkylamino of 1 to 6 carbon atoms, alkanol of 1 to 6 carbon atoms, aralkyl, aryl of 6 to 10 carbon atoms and 3 to 10 membered heterocycle;
n is 2, and
J is a counter ion.

13. The compound for use according to claim 11, wherein wherein R₁ and R₂ are independently selected from methyl, ethyl, n-propyl, or i-propyl;
Xa is CH;
Ya is hydrogen;
n is 2;
J is a halogen.

14. The compound for use according to claim 11, wherein said compound has the formula: 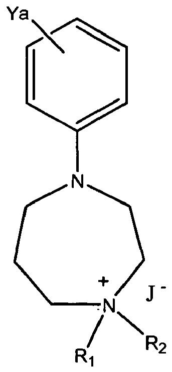 wherein R₁ and R₂ are independently selected from methyl, ethyl, n-propyl, or i-propyl;
Ya is hydrogen;
J is a halogen.

15. The compound for use according to claim 11, wherein said compound has the formula:

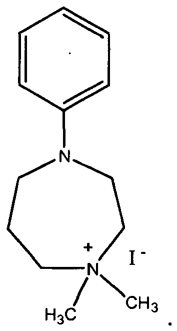

16. The compound for use according to claim 11, wherein R₁ and R₂ are CH₂CH₃, Xa is CH, Ya is H and n is 2.

17. The compound for use according to claim 11, wherein wherein said compound has the formula:

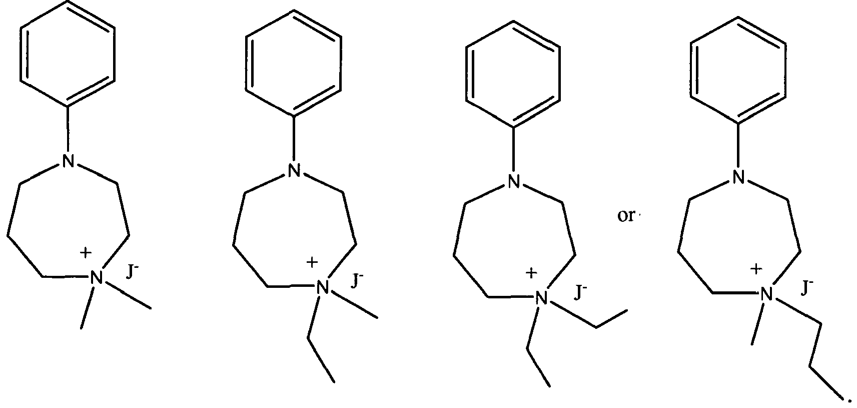

18. The compound for use according to claim any one of claims 11 to 17, wherein said pulmonary inflammatory disease is asthma.

19. The compound for use according to any one of claims claim 11 to 18, wherein said compound is administered by direct injection or infusion, intratracheal/nasal administration, intraocularly, transdermally, orally, parenteraly, topically or by inhalation.

20. The compound for use according to claim 19, wherein said compound is administered orally, topically or by inhalation.

21. A compound according to any one of claims 1-6, for use in inducing airway smooth muscle relaxation and bronchodilation.

22. A compound according to any one of claims 1-6, for use in inducing agonistic response in a pulmonary cell nicotinic receptor.

## Patentansprüche

1. Verbindung der Formel: 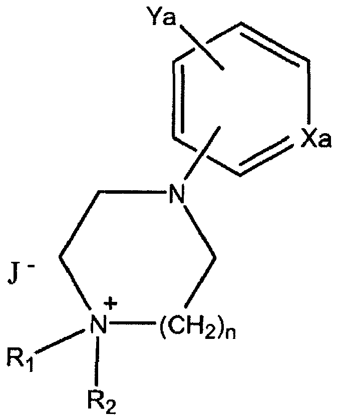 wobei R₁ und R₂ unabhängig Alkyl mit 1 bis 10 Kohlenstoffatomen sind,
Xa CH oder N ist,
Ya ein oder mehrere Substituenten ist, ausgewählt aus Wasserstoff, Halogen, Amidino, Amido, Azido, Cyano, Guanido, Hydroxyl, Nitroso, Harnstoff, Sulfat, Sulfit, Sulfonat, Sulfonamid, Phosphat, Phosphonat, Acyl, Acyloxy, Alkyl mit 1 bis 10 Kohlenstoffatomen, Alkoxy mit 1 bis 10 Kohlenstoffatomen, Alkylthio mit 1 bis 10 Kohlenstoffatomen, Alkylamino mit 1 bis 10 Kohlenstoffatomen, Alkanol mit 1 bis 10 Kohlenstoffatomen, Aralkyl, Aryl mit 6 bis 10 Kohlenstoffatomen und 3- bis 10-gliedrigem Heterocyclus,
n 2 ist;
J ein Gegenion ist.

2. Verbindung nach Anspruch 1, wobei R₁ und R₂ unabhängig aus Methyl, Ethyl, n-Propyl oder i-Propyl ausgewählt sind;
Xa CH ist;
Ya Wasserstoff ist;
n 2 ist;
J ein Halogen ist.

3. Verbindung nach Anspruch 1 mit der Formel:

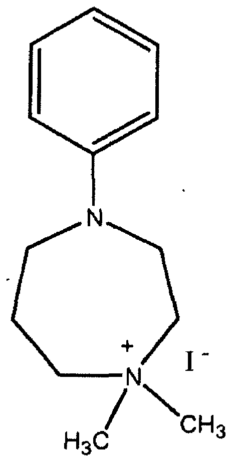

4. Verbindung nach Anspruch 1, wobei R₁ und R₂ CH₂CH₃ sind, Xa CH ist, Ya H ist und n 2 ist.

5. Verbindung nach Anspruch 1, wobei die Verbindung die folgende Formel aufweist:

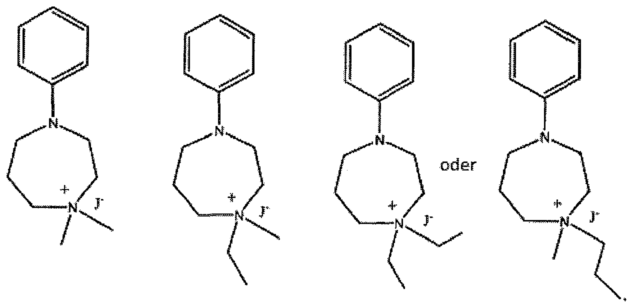

6. Verbindung nach einem der Ansprüche 1 bis 5, wobei J Fluorid, Chlorid, Bromid, Iodid, Sulfat oder Sulfonat ist.

7. Pharmazeutische Zusammensetzung zur Behandlung von entzündlichen Lungenerkrankungen, die eine Verbindung nach einem der Ansprüche 1 bis 6 und einen pharmazeutisch akzeptablen Exzipienten umfasst.

8. Pharmazeutische Zusammensetzung nach Anspruch 7, wobei die entzündlichen Lungenerkrankungen aus der Gruppe, bestehend aus Asthma, chronischer obstruktiver Lungenerkrankung (COPD), interstitieller pulmonaler Fibrose (IPF), Sarkoidose, Hypersensitivitätspneumonitis (HP), chronischer HP und Bronchiolitis obliterans mit organisierender Pneumonie (BOOP), ausgewählt sind.

9. Pharmazeutische Zusammensetzung nach Anspruch 7 oder 8, ferner umfassend ein oder mehrere therapeutische Mittel, ausgewählt aus einem Bronchodilatator, einer entzündungshemmenden Therapie, einem Leukotrienrezeptorantagonisten und Phosphodiesterasehemmern.

10. Pharmazeutische Zusammensetzung nach einem der Ansprüche 7 bis 9, wobei die entzündlichen Lungenerkrankungen Asthma sind.

11. Verbindung zur Verwendung bei der Behandlung oder Prävention von entzündlichen Lungenerkrankungen, ausgewählt aus der Gruppe, bestehend aus Asthma, chronischer obstruktiver Lungenerkrankung (COPD), interstitieller pulmonaler Fibrose (IPF), Sarkoidose, Hypersensitivitätspneumonitis (HP), chronischer HP und Bronchiolitis obliterans mit organisierender Pneumonie (BOOP), wobei die Verbindung die folgende Formel aufweist: 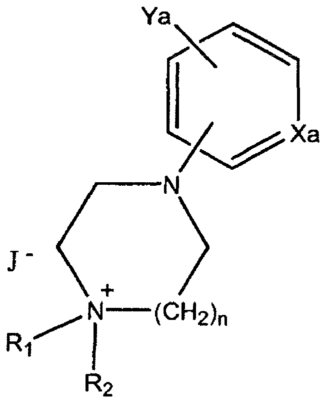 wobei R₁ und R₂ unabhängig Alkyl mit 1 bis 10 Kohlenstoffatomen sind,
Xa CH oder N ist,
Ya ein oder mehrere Substituenten ist, ausgewählt aus Wasserstoff, Halogen, Amino, Amidino, Amido, Azido, Cyano, Guanido, Hydroxyl, Nitro, Nitroso, Harnstoff, Sulfat, Sulfit, Sulfonat, Sulfonamid, Phosphat, Phosphonat, Acyl, Acyloxy, Alkyl mit 1 bis 10 Kohlenstoffatomen, Alkoxy mit 10 bis 10 Kohlenstoffatomen, Alkylthio mit 1 bis 10 Kohlenstoffatomen, Alkylamino mit 1 bis 10 Kohlenstoffatomen, Alkanol mit 1 bis 10 Kohlenstoffatomen, Aralkyl, Aryl mit 6 bis 10 Kohlenstoffatomen und 3- bis 10-gliedrigem Heterocyclus,
n 2 ist;
J ein Gegenion ist.

12. Verbindung zur Verwendung nach Anspruch 23, wobei R₁ und R₂ unabhängig Alkyl mit 1 bis 10 Kohlenstoffatomen sind,
Xa CH oder N ist,
Ya ein oder mehrere Substituenten ist, ausgewählt aus Wasserstoff, Halogen, Amino, Amidino, Amido, Azido, Cyano, Guanido, Hydroxyl, Nitro, Nitroso, Harnstoff, Sulfat, Sulfit, Sulfonat, Sulfonamid, Phosphat, Phosphonat, Acyl, Acyloxy, Alkyl mit 1 bis 6 Kohlenstoffatomen, Alkoxy mit 6 bis 6 Kohlenstoffatomen, Alkylthio mit 1 bis 6 Kohlenstoffatomen, Alkylamino mit 1 bis 6 Kohlenstoffatomen, Alkanol mit 1 bis 10 Kohlenstoffatomen, Aralkyl, Aryl mit 6 bis 10 Kohlenstoffatomen und 3- bis 10-gliedrigem Heterocyclus;
n 2 ist, und
J ein Gegenion ist.

13. Verbindung zur Verwendung nach Anspruch 11, wobei R₁ und R₂ unabhängig aus Methyl, Ethyl, n-Propyl oder i-Propyl ausgewählt sind;
Xa CH ist;
Ya Wasserstoff ist;
n 2 ist;
J ein Halogen ist.

14. Verbindung zur Verwendung nach Anspruch 11, wobei die Verbindung die folgende Formel aufweist: 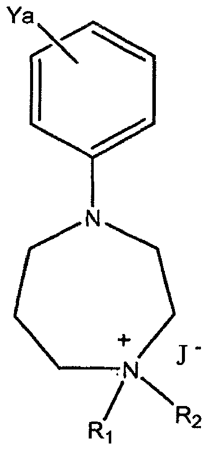 wobei R₁ und R₂ unabhängig aus Methyl, Ethyl, n-Propyl oder i-Propyl ausgewählt sind;
Ya Wasserstoff ist;
J ein Halogen ist.

15. Verbindung zur Verwendung nach Anspruch 11, wobei die Verbindung die folgende Formel aufweist:

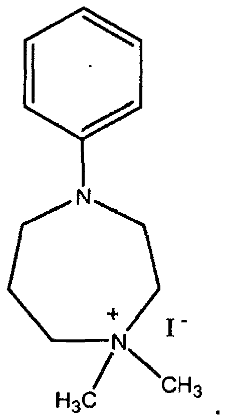

16. Verbindung zur Verwendung nach Anspruch 11, wobei R₁ und R₂ CH₂CH₃ sind, Xa CH ist, Ya H ist und n 2 ist.

17. Verbindung zur Verwendung nach Anspruch 11, wobei die Verbindung die folgende Formel aufweist:

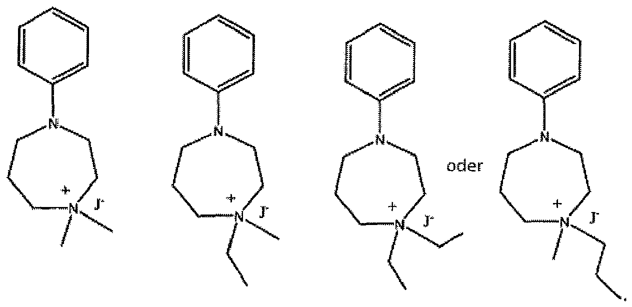

18. Verbindung zur Verwendung nach einem der Ansprüche 11 bis 17, wobei die entzündlichen Lungenerkrankungen Asthma sind.

19. Verbindung zur Verwendung nach einem der Ansprüche 11 bis 18, wobei die Verbindung durch direkte Injektion oder Infusion, intratracheale/nasale Verabreichung, intraokular, transdermal, oral, parenteral, topisch oder durch Inhalation verabreicht wird.

20. Verbindung zur Verwendung nach Anspruch 19, wobei die Verbindung oral, topisch oder durch Inhalation verabreicht wird.

21. Verbindung nach einem der Ansprüche 1 bis 6 zur Verwendung beim Induzieren einer Entspannung glatter Muskel der Atemwege und Bronchodilatation.

22. Verbindung nach einem der Ansprüche 1 bis 6 zur Verwendung beim Induzieren einer agonistischen Antwort in einem nikotinischen Lungenzellrezeptor.

## Revendications

1. Composé de formule : 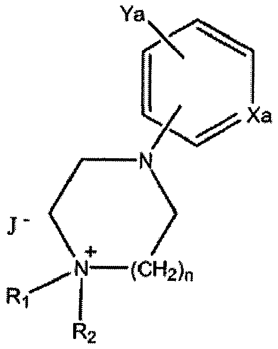 dans laquelle
R₁ et R₂ sont indépendamment des radicaux alkyle ayant de 1 à 10 atomes de carbone,
Xa est CH ou N,
Ya est un ou plusieurs substituants choisis parmi l'hydrogène, les halogènes et les radicaux amidino, amido, azido, cyano, guanido, hydroxyle, nitroso, urée, sulfate, sulfite, sulfonate, sulfonamide, phosphate, phosphonate, acyle, acyloxy, alkyle ayant de 1 à 10 atomes de carbone, alcoxy ayant de 1 à 10 atomes de carbone, alkylthio ayant de 1 à 10 atomes de carbone, alkylamino ayant de 1 à 10 atomes de carbone, alcanol ayant de 1 à 10 atomes de carbone, aralkyle, aryle ayant de 6 à 10 atomes de carbone et hétérocycle à 3 à 10 chaînons,
n vaut 2,
J est un contre-ion.

2. Composé selon la revendication 1, dans lequel
R₁ et R₂ sont indépendamment choisis parmi méthyle, éthyle, n-propyle et isopropyle ;
Xa est CH ;
Ya est l'hydrogène ;
n vaut 2 ;
J est un halogène.

3. Composé selon la revendication 1, de formule :

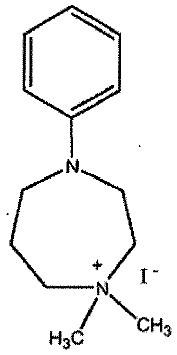

4. Composé selon la revendication 1, dans lequel R₁ et R₂ sont CH₂CH₃, Xa est CH, Ya est H et n vaut 2.

5. Composé selon la revendication 1, lequel composé est de formule :

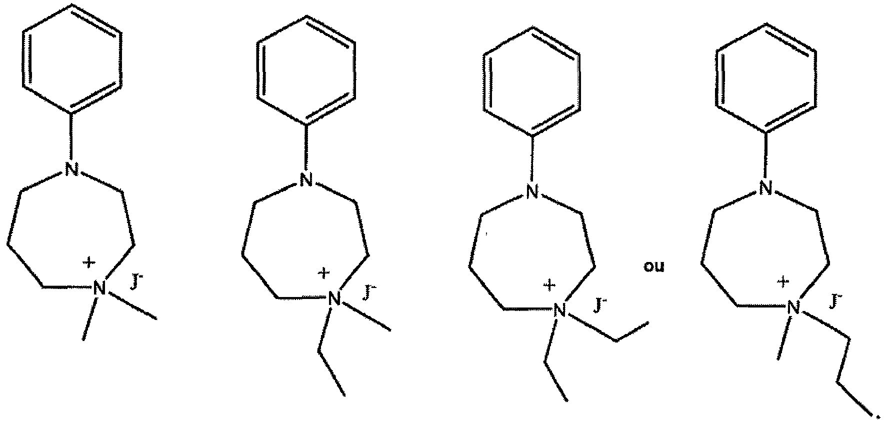

6. Composé selon l'une quelconque des revendications 1 à 5, dans lequel J est un fluorure, chlorure, bromure, iodure, sulfate ou sulfonate.

7. Composition pharmaceutique pour traiter des maladies inflammatoires pulmonaires comprenant un composé selon l'une quelconque des revendications 1 à 6 et un excipient pharmaceutiquement acceptable.

8. Composition pharmaceutique selon la revendication 7, dans laquelle les maladies inflammatoires pulmonaires sont choisies dans le groupe constitué par l'asthme, la broncho-pneumopathie chronique obstructive (BPCO), la fibrose pulmonaire alvéolaire (FPA), la sarcoïdose, la pneumopathie d'hypersensibilité (PH), la PH chronique et la bronchiolite oblitérante avec organisation pneumonique (BOOP).

9. Composition pharmaceutique selon la revendication 7 ou 8, comprenant en outre un ou plusieurs agents thérapeutiques choisis parmi un bronchodilatateurs, un traitement anti-inflammatoire, un antagoniste de récepteur de leucotriène et un inhibiteur de phosphodiestérase.

10. Composition pharmaceutique selon l'une quelconque des revendications 7 à 9, dans laquelle ladite maladie inflammatoire pulmonaire est l'asthme.

11. Composé pour utilisation dans le traitement ou la prévention de maladies inflammatoires pulmonaires choisies dans le groupe constitué par l'asthme, la broncho-pneumopathie chronique obstructive (BPCO), la fibrose pulmonaire alvéolaire (FPA), la sarcoïdose, la pneumopathie d'hypersensibilité (PH), la PH chronique et la bronchiolite oblitérante avec organisation pneumonique (BOOP), lequel composé est de formule : 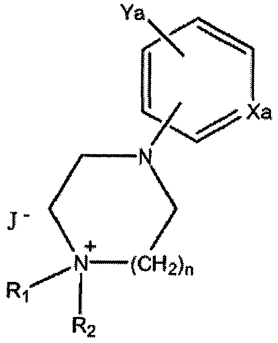 dans laquelle
R₁ et R₂ sont indépendamment des radicaux alkyle ayant de 1 à 10 atomes de carbone,
Xa est CH ou N,
Ya est un ou plusieurs substituants choisis parmi l'hydrogène, les halogènes et les radicaux amino, amidino, amido, azido, cyano, guanido, hydroxyle, nitro, nitroso, urée, sulfate, sulfite, sulfonate, sulfonamide, phosphate, phosphonate, acyle, acyloxy, alkyle ayant de 1 à 10 atomes de carbone, alcoxy ayant de 1 à 10 atomes de carbone, alkylthio ayant de 1 à 10 atomes de carbone, alkylamino ayant de 1 à 10 atomes de carbone, alcanol ayant de 1 à 10 atomes de carbone, aralkyle, aryle ayant de 6 à 10 atomes de carbone et hétérocycle à 3 à 10 chaînons,
n vaut 2,
J est un contre-ion.

12. Composé pour utilisation selon la revendication 23, dans lequel
R₁ et R₂ sont indépendamment des radicaux alkyle ayant de 1 à 10 atomes de carbone,
Xa est CH ou N,
Ya est un ou plusieurs substituants choisis parmi l'hydrogène, les halogènes et les radicaux amino, amidino, amido, azido, cyano, guanido, hydroxyle, nitro, nitroso, urée, sulfate, sulfite, sulfonate, sulfonamide, phosphate, phosphonate, acyle, acyloxy, alkyle ayant de 1 à 6 atomes de carbone, alcoxy ayant de 1 à 6 atomes de carbone, alkylthio ayant de 1 à 6 atomes de carbone, alkylamino ayant de 1 à 6 atomes de carbone, alcanol ayant de 1 à 6 atomes de carbone, aralkyle, aryle ayant de 6 à 10 atomes de carbone et hétérocycle à 3 à 10 chaînons,
n vaut 2, et
J est un contre-ion.

13. Composé pour utilisation selon la revendication 11, dans lequel
R₁ et R₂ sont indépendamment choisis parmi méthyle, éthyle, n-propyle et isopropyle ;
Xa est CH ;
Ya est l'hydrogène ;
n vaut 2 ;
J est un halogène.

14. Composé pour utilisation selon la revendication 11, lequel composé est de formule : 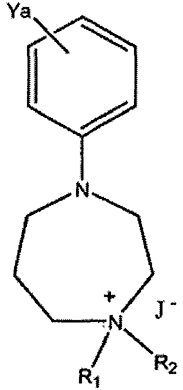 dans laquelle
R₁ et R₂ sont indépendamment choisis parmi méthyle, éthyle, n-propyle et isopropyle ;
Ya est l'hydrogène ;
J est un halogène.

15. Composé pour utilisation selon la revendication 11, lequel composé est de formule :

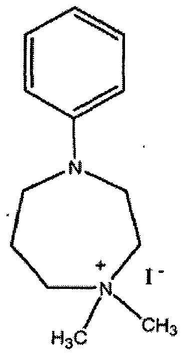

16. Composé pour utilisation selon la revendication 11, dans lequel R₁ et R₂ sont CH₂CH₃, Xa est CH, Ya est H et n vaut 2.

17. Composé pour utilisation selon la revendication 11, lequel composé est de formule :

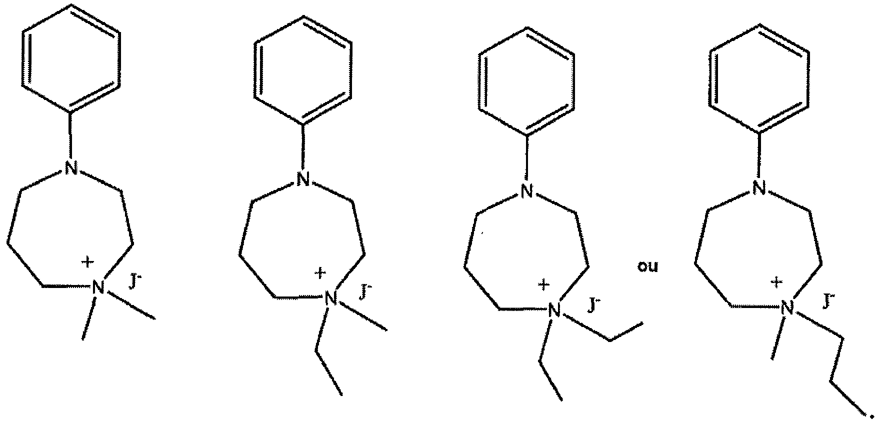

18. Composé pour utilisation selon l'une quelconque des revendications 11 à 17, dans lequel ladite maladie inflammatoire pulmonaire est l'asthme.

19. Composé pour utilisation selon l'une quelconque des revendications 11 à 18, lequel composé est administré par perfusion ou injection directe, par voie intratrachéale/nasale, intraoculaire, transdermique, orale, parentérale, topique, ou par inhalation.

20. Composé pour utilisation selon la revendication 19, lequel composé est administré par voie orale, par voie topique ou par inhalation.

21. Composé pour utilisation selon l'une quelconque des revendications 1 à 6, pour utilisation dans l'induction d'une bronchodilatation et d'une relaxation des muscles lisses des voies aériennes.

22. Composé pour utilisation selon l'une quelconque des revendications 1 à 6, pour utilisation dans l'induction d'une réponse agoniste dans un récepteur nicotinique de cellule pulmonaire.
